(19) Europäisches Patentamt
European Patent Office
Office européen des brevets

(11) Veröffentlichungsnummer: **0 352 530 A2**

(12) **EUROPÄISCHE PATENTANMELDUNG**

(21) Anmeldenummer: **89112510.6**

(22) Anmeldetag: **08.07.89**

(51) Int. Cl.4: **C07D 405/12 , C07D 409/12 , C07D 417/12 , A01N 43/40 , A01N 43/54 , A01N 43/78 , C07D 307/91 , C07D 333/76**

(30) Priorität: **23.07.88 DE 3825173**

(43) Veröffentlichungstag der Anmeldung: **31.01.90 Patentblatt 90/05**

(84) Benannte Vertragsstaaten: **AT BE CH DE ES FR GB IT LI NL**

(71) Anmelder: **BAYER AG**

**D-5090 Leverkusen 1 Bayerwerk(DE)**

(72) Erfinder: **Alig, Bernd, Dr.**
**Gartenstrasse 2**
**D-5630 Königswinter 21(DE)**
Erfinder: **Stendel, Wilhelm, Dr.**
**In den Birken 55**
**D-5600 Wuppertal 1(DE)**
Erfinder: **Londershausen, Michael, Dr.**
**Galileistrasse 11**
**D-4006 Erkrath(DE)**

(54) **Substituierte tricyclische Verbindungen, Verfahren zu ihrer Herstellung und ihre Verwendung als Insektizide.**

(57) Die vorliegende Erfindung betrifft neue, substituierte tricyclische Verbindungen der Formel I

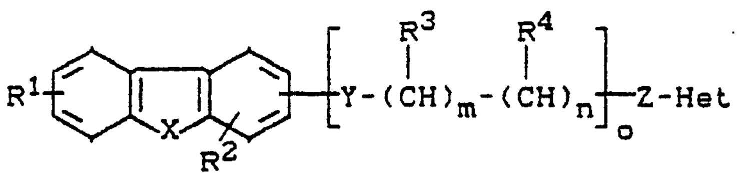

I

in welcher
X für O, S, NH, $-CH_2-$, $-CHCH_3-$, $-C(CH_3)_2-$steht,
Y für O oder S steht,
Z für O oder S steht,
$R^1$ für gleiche oder verschiedene Reste aus der Gruppe Wasserstoff, Halogen, Alkyl, Alkoxy, Halogenalkyl steht,
$R^2$ für gleiche oder verschiedene Reste aus der Gruppe Wasserstoff, Halogen, Alkyl, Alkenyl, Halogen alkyl, Alkoxy, Alkylthio, Halogenalkoxy, Halogenalkylthio, steht,
$R^3$ für Wasserstoff, Alkyl, Halogenalkyl, Alkoxyalkyl, Alkenoxyalkyl, Alkenyl, Alkinyl steht,
$R^4$ für die bei $R^3$ angegebenen Reste steht,
$R^3$ und $R^4$ gemeinsam mit den angrenzenden C-Atomen für gesättigte oder ungesättigte 5- bis 7-gliedrige carbocyclische Ringe stehen können,
m für 1 oder 2 steht,
n für 1 oder 2 steht,
o für 1, 2 oder 3 steht und
Het für gegebenenfalls substituiertes Heteroaryl steht, das nicht über das Heteroatom an den übrigen Rest gebunden ist,

EP 0 352 530 A2

Verfahren zu ihrer Herstellung sowie ihre Verwendung zur Bekämpfung von Insekten, insbesondere Flöhen.

# Substituierte tricyclische Verbindungen, Verfahren zu ihrer Herstellung und ihre Verwendung als Insektizide

Die vorliegende Erfindung betrifft substituierte tricyclische Verbindungen, Verfahren zu ihrer Herstellung, Zwischenprodukte dafür sowie ihre Verwendung als Insektizide.

1. Es wurden die neuen substituierten tricyclischen Verbindungen der Formel I gefunden

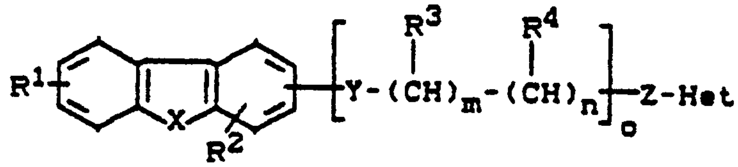

I

in welcher
X für O, S, NH, $-CH_2-$, $-CHCH_3-$, $C(CH_3)_2$-steht,
Y für O oder S steht,
Z für O oder S steht,
$R^1$ für gleiche oder verschiedene Reste aus der Gruppe Wasserstoff, Halogen, Alkyl, Alkoxy, Halogenalkyl steht,
$R^2$ für gleiche oder verschiedene Reste aus der Gruppe Wasserstoff, Halogen, Alkyl, Alkenyl, Halogenalkyl, Alkoxy, Alkylthio, Halogenalkoxy, Halogenalkylthio, steht,
$R^3$ für Wasserstoff, Alkyl, Halogenalkyl, Alkoxyalkyl, Alkenoxyalkyl, Alkenyl, Alkinyl steht,
$R^4$ für die bei $R^3$ angegebenen Reste steht,
$R^3$ und $R^4$ gemeinsam mit den angrenzenden C-Atomen für gesättigte oder ungesättigte 5- bis 7-gliedrige carbocyclische Ringe stehen können,
m für 1 oder 2 steht,
n für 1 oder 2 steht,
o für 1, 2 oder 3 steht und
Het für gegebenenfalls substituiertes Heteroaryl steht, das nicht über das Heteroatom an den übrigen Rest gebunden ist.
sowie deren Salze mit Säuren.

2. Es wurden Verfahren zur Herstellung der substituierten tricyclischen Verbindungen der Formel I gefunden

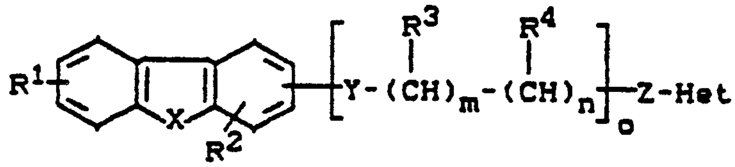

I

in welcher
X für O, S, NH, $-CH_2-$, $-CHCH_3-$, $-C(CH_3)_2$-steht,
Y für O oder S steht,
Z für O oder S steht,
$R^1$ für gleiche oder verschiedene Reste aus der Gruppe Wasserstoff, Halogen, Alkyl, Alkoxy, Halogenalkyl steht,
$R^2$ für gleiche oder verschiedene Reste aus der Gruppe Wasserstoff, Halogen, Alkyl, Alkenyl, Halogenalkyl, Alkoxy, Alkylthio, Halogenalkoxy, Halogenalkylthio, steht,
$R^3$ für Wasserstoff, Alkyl, Halogenalkyl, Alkoxyalkyl, Alkenoxyalkyl, Alkenyl, Alkinyl steht,
$R^4$ für die bei $R^3$ angegebenen Reste steht,
$R^3$ und $R^4$ gemeinsam mit den angrenzenden C-Atomen für gesättigte oder ungesättigte 5- bis 7-gliedrige carbocyclische Ringe stehen können,

m für 1 oder 2 steht,
n für 1 oder 2 steht,
o für 1, 2 oder 3 steht und
Het für gegebenenfalls substituiertes Heteroaryl steht, das nicht über das Heteroatom an den übrigen Rest gebunden ist,
die dadurch gekennzeichnet sind, daß man

a) Verbindungen der Formel II

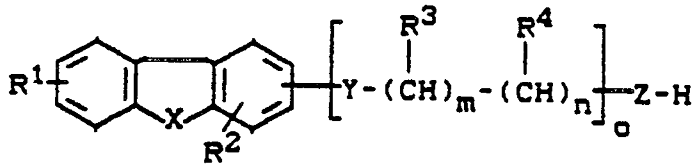

II

in welcher
$R^1$, $R^2$, $R^3$, $R^4$, X, Y, Z, m, n, o die oben angegebene Bedeutung haben,
mit Halogenheterocyclen der Formel III
Hal-Het III
in welcher
Hal für Halogen steht und
Het die oben angegebene Bedeutung hat,
umsetzt, oder

b) Verbindungen der Formel IV

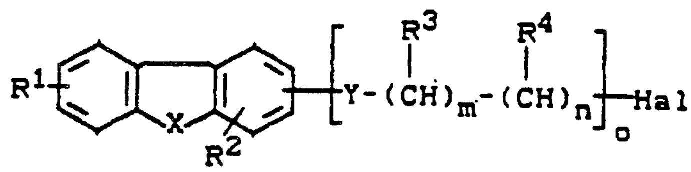

IV

in welcher
$R^1$, $R^2$, $R^3$, $R^4$, X, Y, m, n, o die oben angegebene Bedeutung haben, und
Hal für Halogen steht,
mit Hydroxy oder Thio-heterocyclen der Formel V
H-O(S)-Het V
in welcher
Het die oben angegebene Bedeutung hat,
umsetzt, oder

c) für den Fall, daß in Formel I $R^2$ für Halogen steht, man Verbindungen der Formel I, in welcher $R^2$ für Wasserstoff steht und die übrigen Substituenten die oben angegebenen Bedeutungen haben, mit Halogenierungsmitteln umsetzt.

3. Die Verbindungen der Formel II

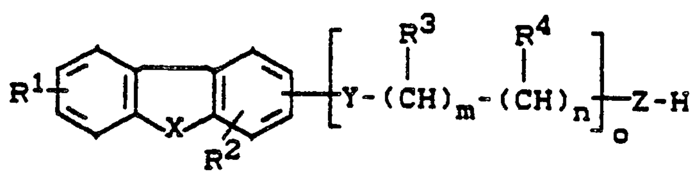

II

in welcher
$R^1$, $R^2$, $R^3$, $R^4$, X, Y, Z, m, n, o die oben angegebene Bedeutung haben,
sind neu.

4. Sie werden hergestellt, indem man Verbindungen der Formel VI

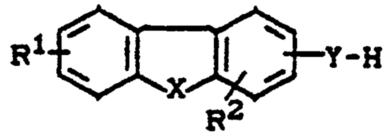

VI

in welcher $R^1$, $R^2$, X, Y die oben angegebene Bedeutung haben, mit Verbindungen der Formeln VII oder VIII

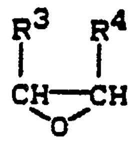

VII

in welcher $R^3$ und $R^4$ die oben angegebene Bedeutung haben,

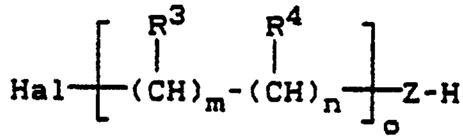

VIII

in welcher Hal, $R^3$, $R^4$, Z, m, n, o die oben angegebene Bedeutung haben, umsetzt.

5. Die Verbindungen der Formel IV

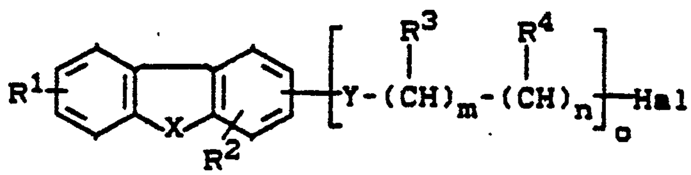

IV

in welcher $R^1$, $R^2$, $R^3$, $R^4$, X, Y, Z, m, n, o die oben angegebene Bedeutung haben, und Hal für Halogen steht, sind neu.

6. Sie werden hergestellt, indem man

a) Verbindungen der Formel VI

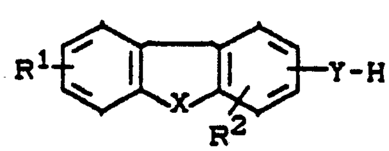

VI

in welcher $R^1$, $R^2$, X, Y die oben angegebene Bedeutung haben, mit 1,n-Dihalogenverbindungen der Formel XI

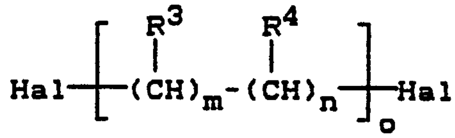

IX

in welcher
Hal, $R^3$, $R^4$, m, n, o die oben angegebene Bedeutung haben,
umsetzt, oder indem man
b) Verbindungen der Formel II

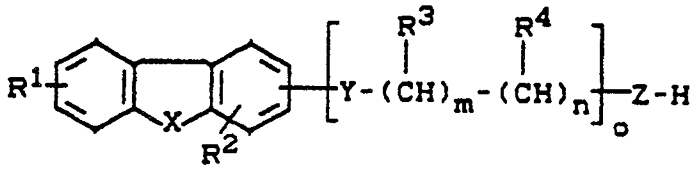

II

in welcher
$R^1$, $R^2$, $R^3$, $R^4$, X, Y, Z, m, n, o die oben angegebene Bedeutung haben,
mit Halogenierungsmitteln umsetzt.

Die Verbindungen der Formel I sowie ihre Salze mit Säuren eignen sich zur Bekämpfung von Insekten. Sie lassen sich besonders bevorzugt zur Bekämpfung von Flöhen einsetzen, insbesondere gegen Pulex irritans, Xenopsylla cheopis, Ctenocephalides canis, Ctenocephalides felis, Ceratophyllus gallinae, Echidnophaga gallinacea, Tunga penetrans. Die Verbindungen der Formel I besitzen die Entwicklung von Insekten hemmende Eigenschaften. Sie können daher gegen alle oder einzelne Entwicklungsstadien von Insekten, insbesondere der Flöhe eingesetzt werden.

Bevorzugt sind Verbindungen der Formel I,
in welcher
X für O, S, $CH_2$ steht,
Y für O steht,
Z für O steht,
$R^1$ für gleiche oder verschiedene Reste aus der Gruppe Wasserstoff, Halogen wie Chlor steht,
$R^2$ für gleiche oder verschiedene Reste aus der Gruppe Wasserstoff, Chlor, Brom, $C_{1-4}$-Alkyl wie Methyl, Ethyl, i-Propyl, t-Butyl, $C_{2-4}$-Alkenyl wie Allyl, $C_{1-4}$-Halogenalkyl wie Trifluormethyl, $C_{1-4}$-Alkoxy, $C_{1-4}$-Halogenalkoxy wie Trifluormethoxy, steht,
$R^3$ für Wasserstoff, $C_{1-4}$-Alkyl wie Methyl, Ethyl steht,
$R^4$ für Wasserstoff, $C_{1-4}$-Alkyl wie Methyl, Ethyl, Propyl, Butyl, $C_{1-4}$-Halogenalkyl wie Trifluormethyl, $C_{1-4}$-Alkoxy-$C_{1-4}$-alkyl wie Methoxymethyl, n- oder t-Butoxymethyl, i-Propoxymethyl, $C_{2-4}$-Alkenyl wie Allyl, $C_{2-4}$-Alkenoxy-$C_{1-4}$-alkyl wie Alkyloxymethyl steht,
$R^3$ und $R^4$ gemeinsam mit den angrenzenden C-Atomen für Cyclopentyl oder Cyclohexyl stehen können,
m für 1 oder 2 steht,
n für 1 steht,
o für 1, 2 oder 3 steht,
Het für Pyridyl, Pyrimidinyl, Pyridazinyl, Pyrazinyl, Thiazolyl, Benzthiazolyl, die gegebenenfalls durch Halogen, $C_{1-4}$-Alkyl wie Methyl, Ethyl, $C_{1-4}$-Halogenalkyl wie Trifluormethyl, Amino, Acylamino wie z.B. substituiertes Phenylureido, Morpholinyl, Dimethylmorpholinyl, Piperazinyl, N-Alkylpiperazinyl, substituiert sein können, steht.

Besonders bevorzugt sind Verbindungen der Formel I,
in welcher
X für O, S steht,
Y für O steht,
Z für O steht,
$R^1$ für Wasserstoff steht,
$R^2$ für Wasserstoff, Halogen, insbesondere Chlor oder Brom, $C_{1-4}$-Alkyl, $C_{2-4}$-Alkenyl, insbesondere Alkyl steht,
$R^3$ für Wasserstoff steht,
$R^4$ für Wasserstoff, $C_{1-6}$-Alkyl, insbesondere Methyl, Ethyl, Propyl, Butyl, $C_{2-4}$-Alkenyl, insbesondere Allyl, $C_{1-4}$-Alkoxy-$C_{1-4}$-alkyl, insbesondere Methoxy-, Ethoxy-, Isopropoxy-, Butoxymethyl, $C_{2-4}$-Alkenoxy-$C_{1-4}$-alkyl, insbesondere Allyloxymethyl steht,
$R^3$ und $R^4$ gemeinsam mit den angrenzenden C-Atomen für Cyclopentyl oder Cyclohexyl stehen,
m für 1 oder 2 steht,
n für 1 oder 2 steht,
o für 1, 2 oder 3 steht,

Het für Pyridyl, Pyrimidinyl, Pyrazinyl, Thiazolyl, die gegebenenfalls durch $C_{1-4}$-Alkyl, $C_{1-4}$-Halogenalkyl, insbesondere Trifluormethyl, Amino, Halogen, insbesondere Chlor, $C_{1-4}$-Alkylureido, insbesondere Methylureido, Arylureido, insbesondere Phenylureido, wobei der Phenylring gegebenenfalls durch Halogen, Alkyl, Alkoxy, Halogenalkyl, Halogenalkoxy, substituiert sein kann, steht.

Im einzelnen seien genannt:

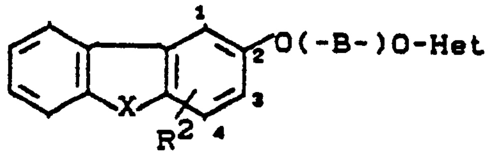

| X | $R^2$ | B | Het |
|---|---|---|---|
| O | 1,3 $Cl_2$ | 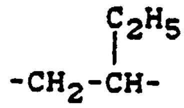 | 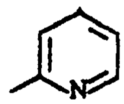 |
| S | H | 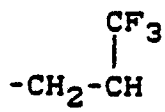 | 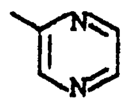 |
| O | 1,3-$Cl_2$ | 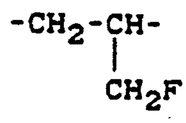 | 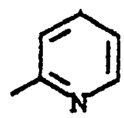 |
| O | 1,3-$Cl_2$ | 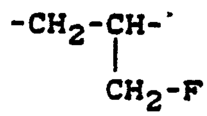 | 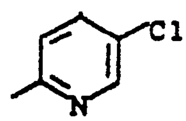 |
| S | 1,3-$Cl_2$ | 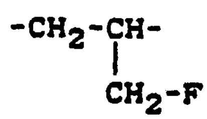 | 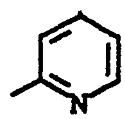 |
| O | 3-Br | 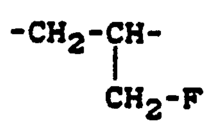 | 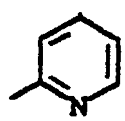 |

| X | $R^2$ | B | Het |
|---|---|---|---|
| $CH_2$ | 1,3-$Cl_2$ | 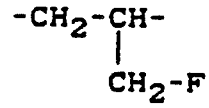 | 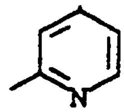 |
| $CH_2$ | H | 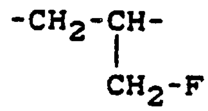 | 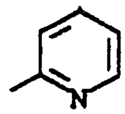 |
| O | H | 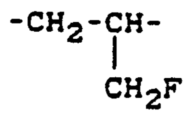 | 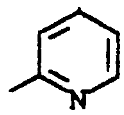 |
| S | H | 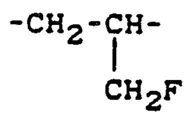 | 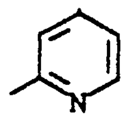 |
| O | 1,3-$Cl_2$ | 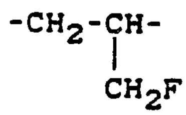 | 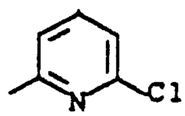 |
| O | 1,3-$Cl_2$ | 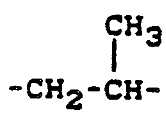 | 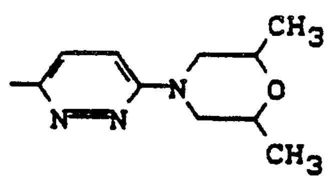 |
| S | 1,3-$Cl_2$ | 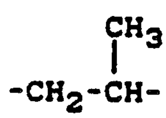 | 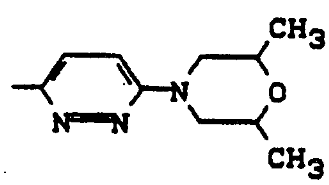 |

| X | $R^2$ | B | Het |
|---|---|---|---|
| $CH_2$ | 1,3-$Cl_2$ | 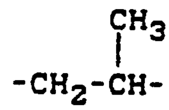 | 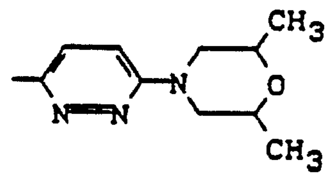 |
| O | H | 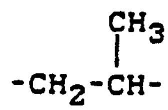 | 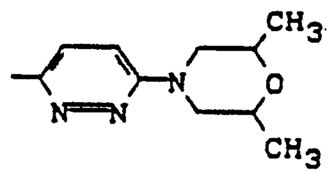 |
| O | 1,3-$Cl_2$ | 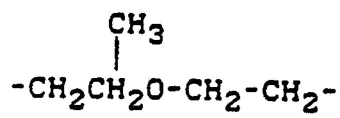 | 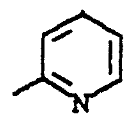 |

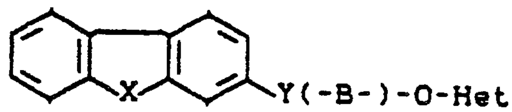

| X | Y | B | Het |
|---|---|---|---|
| O | S | $-CH_2CH_2OCH_2CH_2$ | 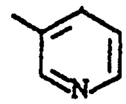 |
| O | O | $CH_2-CH_2-O-CH_2-CH_2$ | 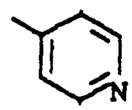 |
| O | S | 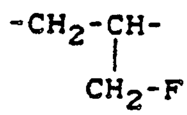 | 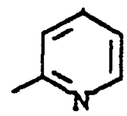 |
| S | O | 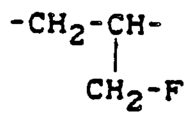 | 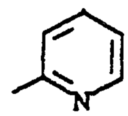 |
| O | O | 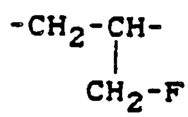 | 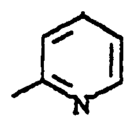 |
| $CH_2$ | S | 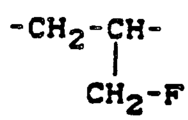 | 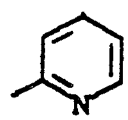 |
| O | O | 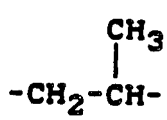 | 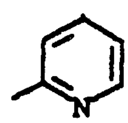 |

| X | Y | B | Het |
|---|---|---|---|
| O | O | 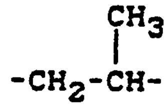 | 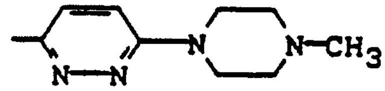 |
| O | O | 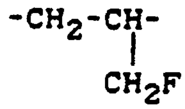 | 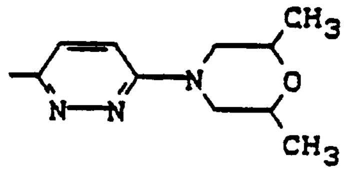 |
| S | O | 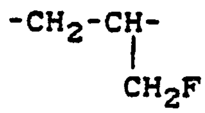 | 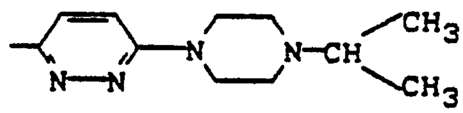 |
| $CH_2$ | O | 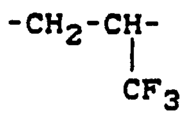 | 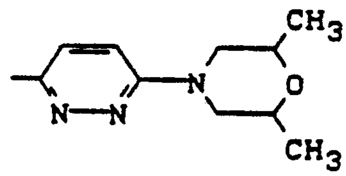 |
| O | O | 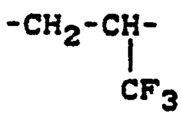 | 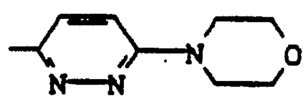 |
| O | O | 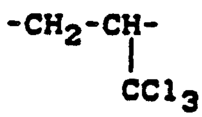 | 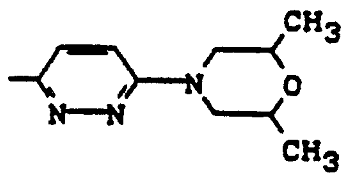 |
| S | O | 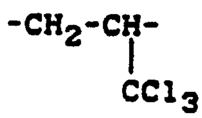 | 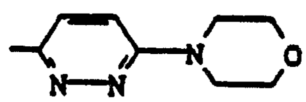 |

Setzt man bei Verfahren 2a zur Herstellung der erfindungsgemäßen Verbindungen als Verbindung der Formel II 2-Hydroxyisopropoxydibenzofuran und als Verbindung der Formel III 3-Chlorpyridin ein, läßt sich der Reaktionsverlauf durch folgendes Formelschema kennzeichnen:

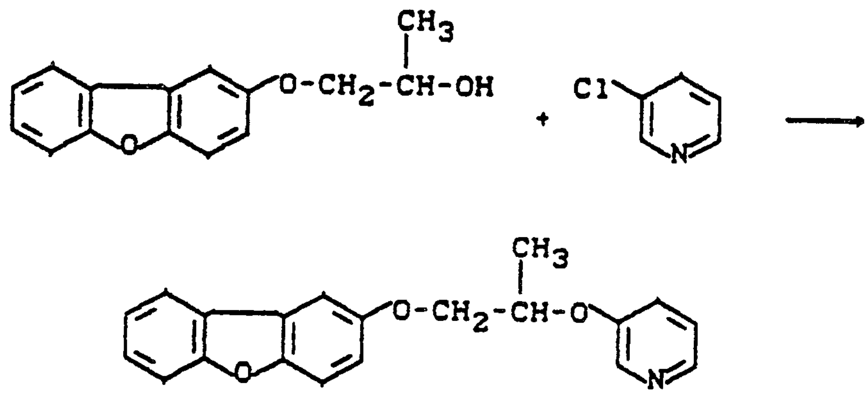

Bevorzugt werden Verbindungen der Formel II und III eingesetzt, in denen $R^1$, $R^2$, $R^3$, $R^4$, X, Y, Z, Het, m, n, o die bei den Verbindungen der Formel I angegebenen bevorzugten oder besonders bevorzugten Bedeutungen besitzen.

Im einzelnen seien folgende Verbindungen der Formel II genannt:

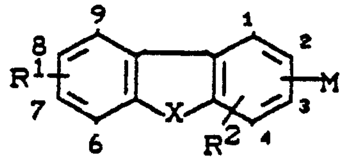

| $R^1$ | X | $R^2$ | M |
|---|---|---|---|
| H | $CH_2$ | 1-Cl | $3 - O-CH_2-CH_2-SH$ |
| 7-Cl | O | H | $2-O-CH_2-CH_2-SH$ |
| 7-Cl | O | H | $2-O-CH_2-CH_2-OH$ |
| 7-Cl | S | H | $2-O-CH_2-CH_2-OH$ |
| H | O | $3-OCH_3$ | $2-O-CH_2-CH-OH$<br>\|<br>$CH_3$ |
| H | O | $3-OCH_3$ | $2-O-CH_2-CH-OH$<br>\|<br>$CF_3$ |
| 7-Cl | $CH-CH_3$ | H | $2-O-CH_2-CH_2-SH$ |
| 7-Cl | O | H | $2-O-CH_2-CH-OH$<br>\|<br>$CH_2F$ |
| 7-Cl | O | H | $3-O-CH_2-CH-OH$<br>\|<br>$CH_2F$ |
| H | S | $3-OCH_3$ | $2-O-CH_2-CH-OH$<br>\|<br>$CH_2F$ |
| 7-Cl | O | H | $3-O-CH_2-CH-SH$<br>\|<br>$CH_2-OCF_3$ |
| 7-Cl | S | H | $2-O-CH_2-CH-OH$<br>\|<br>$CH_2-OCF_3$ |
| H | O | 1-Cl,3-t-Butyl | $2-O-CH-CH_2-OH$<br>\|<br>$CH_3$ |

In einzelnen seien folgende Verbindungen der Formel III genannt: 2-Chlorpyridin, 2-Brompyridin, 2-Chlorpyrimidin, 2-Brompyrimidin, 3-Chlorpyridin, 3-Brompyridin, 2-Chlor-5-trifluormethylpyridin, 2-Brom-5-methylpyridin, 4-Chlorpyridin, 2-Chlorbenzothiazol, 2-Chlorpyrazin, 2-Bromthiazol, 2-Chlor-6-methylpyridin, 5-Brompyrimidin, 2-Bromthiophen, 3-Bromthiophen, 3-Chlor-2,5-dimethylpyrazin, 4-Chlor-2-methylthiopyrimidin, 2-Chlorthiophen, 2,3-Dichlorpyridin, 2,5-Dichlorpyridin, 2,6-Dichlorpyridin, 2,6-Dibrompyridin, 3,5-Dichlorpyridin, 2,4-Dichlor-6-methylpyrimidin, 2,4-Dichlorpyrimidin, 4,6-Dichlor-pyrimidin, 2,6-Difluorpyridin, 4,6-Dichlor-2-methylthiopyrimidin, 2,6-Dichlor-pyrazin, 3,6-Dichlorpyridazin, 3-Chlor-6-N′-Alkylpiperazinyl-pyridazin, 3-Chlor-6-dimethylmorpholinyl-pyridazin.

Die Verbindungen der Formel II sind neu, ihre Herstellung wird weiter unten beschrieben. Die Verbindungen der Formel III sind bekannt.

Die Umsetzung erfolgt durch Erhitzen der Verbindungen II und III in Gegenwart von Verdünnungsmitteln, Basen sowie gegebenenfalls Katalysatoren.

Die Umsetzung erfolgt bei Temperaturen von 0 - 200° C, bevorzugt bei 20 - 100° C, besonders bevorzugt bei Siedetemperatur des Verdünnungsmittels.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl-und Methylisobutylketon, außerdem Ester, wie Essigsäuremethylester und -ethylester, ferner Nitrile, wie z.B. Acetonitril und Propionitril, Benzonitril, Glutarsäuredinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid.

Als Basen kommen anorganische und organische Basen in Frage. Als Basen seien genannt Alkali- und Erdalkalihydroxide, -carbonate, -hydrogencarbonate, -alkoholate, ferner Amide wie insbesondere tertiäre Amine z.B. Trimethylamin, Triethylamin, N-Methylmorpholin, Pyridin, Picoline,. N-Ethylpyrrolidin, Diazabicyclo(4,3,0)-undecan(DBU), 1,4-Diazabicyclo(2,2,2)octan (DABCO), Diazabicyclo(3,2,0)nonen (DBN).

Als Katalysatoren kommen z.B. in Frage Phasentransferkatalysatoren wie Tris-[2-(2-methoxyethoxy)]-ethyl-amin (TDA-1), Benzyltriethylammoniumchlorid (TEBA), 1,4,7,10,13,16-Hexaoxacyclooctadecan (18-Crown-6), Tetrabutylammoniumbromid, Tetrabutylammoniumhydrogensulfat, Tetrabutylammoniumjodid, Methyltrialkyl ($C_8$-$C_{10}$)-ammoniumchlorid (Adogen 464).

Die Verbindungen der Formeln II und III werden in etwa äquimolarem Verhältnis zueinander eingesetzt. Ein Überschuß der einen oder anderen Komponente bringt keinen wesentlichen Vorteil.

Nach erfolgter Umsetzung wird das Verdünnungsmittel abdestilliert und die Verbindungen der Formel I in an sich bekannter Weise isoliert, indem sie z.B. aus dem Rückstand mit einem geeigneten Lösungsmittel z.B. Ether extrahiert werden. Die Verbindungen der Formel I können anschließend in üblicher Weise z.B. durch Destillation gereinigt werden.

Setzt man bei Verfahren 2b zur Herstellung der Verbindungen der Formel I als Verbindung der Formel IV 3-Chlorpropyloxy-dibenzofuran und als Verbindung der Formel V 3-Amino-4-Mercaptopyridin ein, läßt sich der Reaktionsverlauf durch folgendes Formelschema wiedergeben:

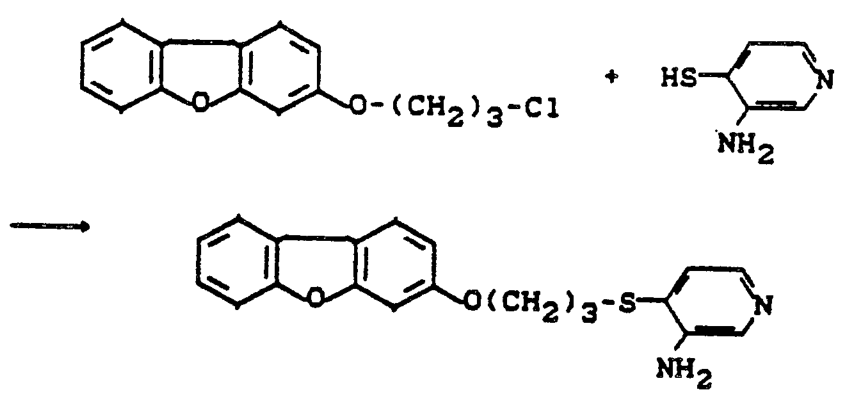

Das Verfahren wird bevorzugt angewendet, wenn die Verbindung der Formel V eine Aminogruppe enthält.

Bevorzugt werden Verbindungen der Formeln IV und V eingesetzt, in denen $R^1$, $R^2$, $R^3$, $R^4$, X, Y, Z, Het, m, n, o die bei den Verbindungen der Formel I angegebenen bevorzugten oder besonders bevorzugten Bedeutungen besitzen.

Im einzelnen seien folgende Verbindungen der Formel IV genannt:

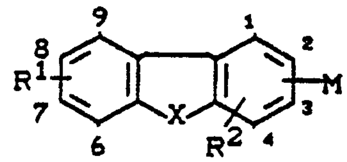

| $R^1$ | X | $R^2$ | M |
|---|---|---|---|
| H | $CH_2$ | 1-Cl | 3 - $O-CH_2-CH_2-Cl$ |
| 7-Cl | O | H | 2-$O-CH_2CH_2-Cl$ |
| H | S | H | 3-$O-CH_2CH_2-Cl$ |
| 7-Cl | O | H | 2-$O-CH_2CH_2CH_2-Cl$ |
| 7-Cl | O | H | 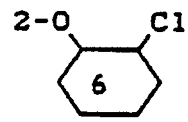 |
| 7-Cl | O | H | 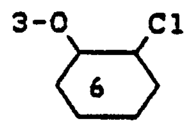 |
| H | O | H | 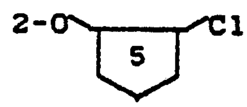 |
| H | $CH_2$ | H | 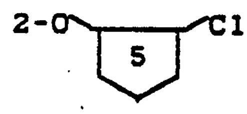 |

| $R^1$ | X | $R^2$ | M |
|---|---|---|---|
| H | O | H | $2\text{-}O\text{-}CH_2CH_2CH_2\text{-}Br$ |
| H | O | H | $2\text{-}O\text{-}CH_2\text{-}CH\text{-}Cl$<br>\|<br>$CH_3$ |
| 7-Cl | O | H | $2\text{-}O\text{-}CH_2\text{-}CH\text{-}Cl$<br>\|<br>$CH_3$ |
| 7-Cl | O | H | $2\text{-}O\text{-}CH_2\text{-}CH\text{-}Cl$<br>\|<br>$C_2H_5$ |

In einzelnen seien folgende Verbindungen der Formel V genannt: 2-Hydroxypyridin, 2-Mercaptopyridin, 2-Hydroxypyrimidin, 3-Hydroxypyridin, 2-Amino-3-hydroxypyridin, 2-Chlor-3-hydroxypyridin, 2,3-Dihydroxypyridin.

Die Verbindungen der Formel IV sind neu, ihre Herstellung wird weiter unten beschrieben. Die Verbindungen der Formel V sind bekannt.

Die Umsetzung erfolgt durch Erhitzen der Verbindungen IV und V in Gegenwart von Verdünnungsmitteln, Basen sowie gegebenenfalls Katalysatoren.

Die Umsetzung erfolgt bei Temperaturen von 0 - 200° C, bevorzugt bei 20 - 100° C, besonders bevorzugt bei Siedetemperatur des Verdünnungsmittels.

Als Verdünnungsmittel kommen alle inerten organischen Lösungsmittel in Frage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Methylenchlorid, Ethylenchlorid, Tetrachlorkohlenstoff, Chlorbenzol und o-Dichlorbenzol, ferner Ether wie Diethyl- und Dibutylether, Glykoldimethylether und Diglykoldimethylether, Tetrahydrofuran und Dioxan, weiterhin Ketone, wie Aceton, Methylethyl-, Methylisopropyl-und Methylisobutylketon, außerdem Ester, wie Essigsäuremethylester und -ethylester, ferner Nitrile, wie z.B. Acetonitril und Propionitril, Benzonitril, Glutarsäure dinitril, darüber hinaus Amide, wie z.B. Dimethylformamid, Dimethylacetamid und N-Methylpyrrolidon, sowie Dimethylsulfoxid, Tetramethylensulfon und Hexamethylphosphorsäuretriamid.

Als Basen kommen anorganische und organische Basen in Frage. Als Basen seien genannt Alkali- und Erdalkalihydroxide, -carbonate, -hydrogencarbonate, -alkoholate, ferner Amine wie insbesondere tertiäre Amine z.B. Trimethylamin, Triethylamin, N-Methylmorpholin, Pyridin, Picoline, N-Ethylpyrrolidin, Diazabicyclo(4,3,0)-undecan(DBU), 1,4-Diazabicyclo(2,2,2)octan (DABCO), Diazabicyclo(3,2,0)nonen (DBN).

Als Katalysatoren kommen z.B. in Frage Phasentransferkatalysatoren wie Methyltrialkyl ($C_8$-$C_{10}$)-ammoniumchlorid (Adogen 464), 1,4,7,10,13,16-Hexaoxacyclooctadecan (18-Crown-6), Tetrabutylammoniumbromid, Tris-[2-(2-methoxyaethoxy)-aethyl]-amin (TDA-1), Benzyltriethylammoniumchlorid (TEBA).

Die Verbindungen der Formeln IV und V werden in etwa äquimolarem Verhältnis zueinander eingesetzt. Ein Überschuß der einen oder anderen Komponente bringt keinen wesentlichen Vorteil.

Nach erfolgter Umsetzung wird das Verdünnungsmittel abdestilliert und die Verbindungen der Formel I in an sich bekannter Weise isoliert, indem sie z.B. aus dem Rückstand mit einem geeigneten Lösungsmittel z.B. Ether extrahiert werden. Die Verbindungen der Formel I können anschließend in üblicher Weise z.B.

durch Destillation gereinigt werden.

Verfahren 2c kann zur Herstellung der Verbindungen der Formel I eingesetzt werden, in denen die Reste $R^2$ für Halogen stehen. Es läßt sich durch folgendes Formelschema darstellen:

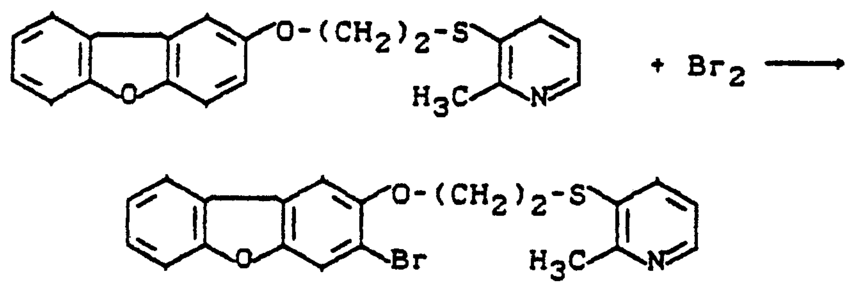

Die Halogenierung wird mit geeigneten Halogenierungsmitteln, gegebenenfalls bei erhöhter Temperatur und in Gegenwart von Verdünnungsmitteln, durchgeführt.

Als Halogenierungsmittel seien genannt: elementares Halogen, insbesondere Brom, Kupfer(II)-halogenide, insbesondere $CuBr_2$, Sulfurylchlorid. Die Halogenierungsmittel werden im allgemeinen in etwa stöchiometrischer Menge zu den Verbindungen der Formel I, in welcher $R^2$ für Wasserstoff steht und die übrigen Substitutionen die oben angegebene Bedeutung haben, eingesetzt. Ein bis zu 5-facher, bevorzugt bis 1,5-facher Überschuß Halogenierungsmittel kann vorteilhaft sein.

Die Halogenierung wird zwischen -10 und +150 °C, bevorzugt zwischen 0 und 100 °C, durchgeführt. Weiter bevorzugt ist das Arbeiten bei Siedetemperatur des Verdünnungsmittels.

Es wird bevorzugt bei Normaldruck gearbeitet. Geeignete Verdünnungsmittel sind gegebenenfalls substituierte aliphatische oder aromatische Kohlenwasserstoffe wie Pentan, Hexan, Heptan, Cyclohexan, Benzin, Benzol, Ligroin, Petrolether, Toluol, Methylenchlorid, Ethylenchlorid, Chloroform, Tetrachlorkohlenstoff, Chlorbenzol, o-Dichlorbenzol. Für die Durchführung der Halogenierung mit Brom oder $CuBr_2$ seien außerdem genannt Alkohole wie Methanol, Ethanol, Ester wie Essigsäurethylester, Eisessig.

Die Halogenierung mit Brom erfolgt bevorzugt in Gegenwart von äquimolaren Mengen Bromwasserstoffsäure.

Die Aufarbeitung der Reaktionsmischung nach Beendigung der Reaktion erfolgt in an sich bekannter Weise.

Setzt man bei Verfahren 4 zur Herstellung der Verbindungen der Formel II als Verbindung der Formel VI 3-Hydroxy-dibenzofuran ein und als Verbindung der Formel VII Propylenoxid, so läßt sich der Reaktionsablauf durch folgendes Formelschema darstellen:

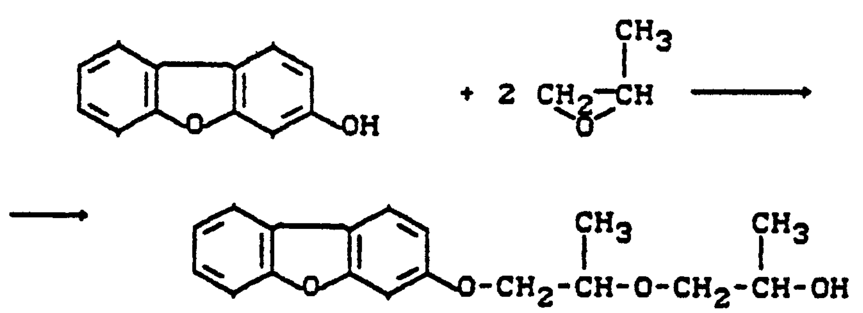

Bevorzugt werden Verbindungen der Formeln VI und VII eingesetzt, in welchen $R^1$, $R^2$, $R^3$, $R^4$, X, Y die bei den Verbindungen der Formel I angegebenen bevorzugten Bedeutungen besitzen.

Im einzelnen seien folgende Verbindungen der Formel VI genannt:

2-Hydroxy-dibenzofuran, 2-Hydroxy-dibenzothiophen, 3-Hydroxy-dibenzofuran, 3-Hydroxy-dibenzothiophen, 2-Hydroxyfluoren, 3-Hydroxyfluoren, 7-Chlor-2-hydroxydibenzofuran, 2-Hydroxy-3-tert.-butyl-dibenzofuran, 2-Hydroxy-3-methoxy-dibenzofuran, 1-Allyl-2-hydroxy-dibenzofuran, 2-Hydroxy-3-allyl-dibenzofuran, 2-Mercaptodibenzofuran, 3-Mercapto-dibenzofuran, 2-Mercapto-dibenzothiophen, 2-Mercapto-fluoren.

Im einzelnen seien folgende Verbindungen der Formel VII genannt:

Propylenoxid, 1-Chlor-2,3-epoxypropan, 1-Fluor-2,3-epoxypropan, 3,3,3-Trifluoro-1,2-epoxypropan, Butyl-2,3-epoxypropylether, 2,3-Epoxypropyl-isopropylether, Allylglycidylether, 1-Brom-2,3-epoxypropan, 1,3-Butadienmonoxid, Cyclopentenoxid, Cyclohexenoxid, Cycloheptenoxid, 1,2-Hexenoxid, 1,2-Decenoxid, 1,2-Dodecenoxid, 1,2-Hexadecenoxid, 2,2-Dimethyl-1-methoxyaethylenoxid, 1,2-Epoxy-3-methoxypropan, 1,5-Hexadienmonoxid, tert.-Butyl-2,3-epoxypropylether, 3-Methoxy-2,2-dimethylenpoxypropan, 1,2-Butylenoxid, 2,2-Dimethyloxiran, 2,3-Dimethyloxiran.

Die Verbindungen der Formeln VI und VII sind bekannt.

Die Umsetzung erfolgt durch Erhitzen einer Mischung der Ausgangsverbindungen in einem Verdünnungsmittel und Waser in Gegenwart von Basen sowie gegebenenfalls in Gegenwart eines Katalysators.

Die Umsetzung erfolgt bei Temperaturen von 0 bis 200 °C, insbesondere bei 50 - 150 °C.

Als Verdünnungsmittel seien genannt:

Pentan, Hexan, Heptan, Cyclohexan, Petrolether, Benzin, Ligroin, Benzol, Toluol, Xylol, Diethylether, Dibutylether, Diglykoldimethylether, Dioxan, Dimethylsulfoxid.

Als Basen seien genannt:

Alkali- und Erdalkalihydroxide, -carbonate.

Als Katalysatoren seien genannt:

Tetrabutylammoniumbromid, Tetrabutylammoniumhydrogensulfat. Tetrabuylammoniumjodid, Triethylbenzylammoniumchlorid (TEBA), Methyl ($C_8$-$C_{10}$)-ammoniumchlorid (Adogen 464), Hexadecyl-tributylphosphoniumbromid, Tetraethylammoniumjodid.

Die Verbindungen VI und VII werden in äquimolarem Verhältnis zueinander eingesetzt, wenn Verbindungen der Formel II erhalten werden sollen, bei denen der Index o = 1 ist. Soll der Index o für 2 oder 3 stehen, werden entsprechende molare Verhältnisse gewählt.

Nach erfolgter Umsetzung erfolgt die Aufarbeitung wie folgt:

Die organische Phase wird von der Wasserphase abgetrennt; nach dem Trocknen über z.B. Natriumsulfat wird vom org.Verdünnungsmittel abdestilliert, aus dem Rückstand werden die Verbindungen der Formel II in üblicher Weise z.B. durch Destillation gereinigt.

Die Umsetzung der Verbindungen der Formel VI mit Verbindungen der Formel VII erfolgt durch Erhitzen einer Mischung der Ausgangsverbindungen in einem Verdünnungsmittel und Wasser in Gegenwart von Basen und Katalysatoren.

Diese Art der Umsetzung wird bevorzugt gewählt, um zu Verbindungen der Formel I zu gelangen, in denen $R^3$ für andere Reste als Wasserstoff steht.

Die Umsetzung erfolgt bei Temperaturen von 0 bis 200 °C, insbesondere bei 50 - 150 °C.

Als Verdünnungsmittel seien genannt:

Pentan, Hexan, Heptan, Cyclohexan, Petrolether, Benzin, Ligroin, Benzol, Toluol, Xylol, Diethylether, Dibutylether, Diglykoldimethylether, Dioxan, Dimethylsulfoxid.

Als Basen seien genannt;

Alkali- und Erdalkalihydroxide-, carbonate.

Als Katalysatoren seien genannt:

Tetrabutylammoniumbromid, Tetrabutylammoniumhydrogensulfat, Tetrabutylammoniumjodid, Triethylbenzylammoniumchlorid (TEBA), Methyl ($C_8$-$C_{10}$)-ammoniumchlorid (Adogen 464), Hexadecyl-tributylphosphoniumbromid, Tetraethylammoniumjodid.

Die Verbindungen VI und VII werden in äquimolarem Verhältnis zueinander eingesetzt, wenn Verbindungen der Formel II erhalten werden sollen, bei denen der Index o = 1 ist. Soll der Index o für 2 oder 3 stehen, werden entsprechende molare Verhältnisse gewählt.

Nach erfolgter Umsetzung erfolgt die Aufarbeitung wie folgt:

Die organische Phase wird von der Wasserphase abgetrennt; nach dem Trocknen über z.B. Natriumsulfat wird vom organischen Verdünnungsmittel abdestilliert, aus dem Rückstand werden die Verbindungen der Formel II in üblicher Weise z.B. durch Destillation gereinigt.

Setzt man bei Verfahren 6 zur Herstellung der Verbindungen der Formel IV als Verbindung der Formel VI 3-Hydroxy-dibenzothiophen ein und als 1,n-Dihalogenverbindung der Formel IX 1,2-Dibrompropan ein, so läßt sich der Reaktionsablauf durch folgendes Formelschema wiedergeben:

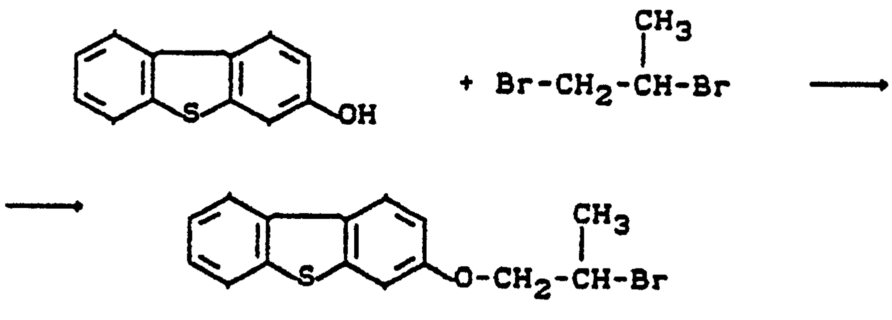

Bevorzugt werden Verbindungen der Formeln VI und IX eingesetzt, in welchen $R^1$, $R^2$, $R^3$, $R^4$, X, Y, m, n, o die oben angegebenen bevorzugten und besonders bevorzugten Bedeutungen haben und Hal für Chlor oder Brom, insbesondere für Brom steht.

Im einzelnen seien die bei Verfahren 4 genannten Verbindungen der Formel VI genannt.

Im einzelnen seien folgende Verbindungen der Formel IX genannt:

1,2-Dibrompropan,
1,3-Dibrompropan,
1,2-Dibrom-3-fluorpropan,
1-Brom-2-Chlorpropan,

Die Umsetzung erfolgt bei Temperaturen von 0 bis 200 °C, insbesondere bei 70 - 130 °C.

Als Verdünnungsmittel seien genannt:

Wasser!, Benzol, Toluol, Diglykoldimethylether, Aceton, Methyl-ethylketon, Methyl-isopropylketon, Acetonitril, Propionitril, Glutarsäuredinitril, N,N-Dimethylformamid.

Als Basen seien genannt:

Alkali- und Erdalkalihydroxide, -carbonate.

Als Katalysatoren seien genannt:

Tetrabutylammoniumbromid, Natriumjodid, Kaliumjodid.

Die Verbindungen VI und IX werden in äquimolarem Verhältnis zueinander eingesetzt, wenn Verbindungen der Formel IV erhalten werden sollen, bei denen der Index o = 1 ist.

Nach erfolgter Umsetzung erfolgt die Aufarbeitung wie folgt:

Das Verdünnungsmittel wird abdestilliert, und die Verbindungen der Formel IV werden isoliert, indem sie z.B. aus dem Rückstand mit einem geeigneten Lösungsmittel wie z.B. Ether extrahiert werden. Die Verbindungen der Formel IV können anschließend in üblicher Weise z.B. durch Destillation gereinigt werden.

Sollen Verbindungen der Formel IV erhalten werden, in denen $R^2$ für Halogen steht, werden gemäß Verfahren 6b Verbindungen der Formel II, in denen $R^2$ für Wasserstoff steht, mit Halogenierungsmitteln umgesetzt. Die Durchführung des Verfahrens erfolgt wie weiter oben bei Verfahren 2c) beschrieben.

Die Anwendung der Wirkstoffe erfolgt direkt oder in Form von geeigneten Zubereitungen, dermal, enteral oder parenteral, oder durch Behandlung der Umgebung oder mit Hilfe wirkstoffhaltiger Formkörper wie z.B. Bänder, Halsbänder, Gliedmaßenbänder, Markierungsvorrichtungen.

Die dermale Anwendung geschieht z.B. in Form des Tauchens (Dippen), Sprühens (Sprayen) oder Aufgießens (pour-on und spot-on) und des Einpuderns.

Zubereitungen zur dermalen Anwendung sind z.B. Lösungen, Suspensions- und Emulsionskonzentrate sowie Mikroemulsionen, die vor Anwendung mit Wasser verdünnt werden, Aufgußformulierungen, Pulver und Stäube, Aerosole und wirkstoffhaltige Formkörper.

Lösungen, Suspensions- und Emulsionskonzentrate sowie Mikroemulsionen die vor der Anwendung mit Wasser verdünnt werden, Pulver, Stäube und Aerosolformulierungen werden zur Behandlung der Umgebung der Haustiere eingesetzt.

Diese Zubereitungen werden in bekannter Weise hergestellt, z.B. durch Vermischen des Wirkstoffs mit Streckmitteln, also z.B. flüssigen Lösungsmitteln, gegebenenfalls unter Verwendung von oberflächenaktiven Mitteln, also Emulgiermitteln und/oder Dispergiermitteln. Im Falle der Benutzung von Wasser als Streckmittel können z.B. auch organische Lösungsmittel als Hilfslösungsmittel verwendet werden.

Zu den flüssigen Verdünnungsmitteln zählen neben Wasser Alkohole wie Methanol, Ethanol, Isopropanol, n-Butanol, Amylalkohol, Octanol;

Glykole wie Propylenglykol, 1,3-Butylenglykol, Ethylglykol, Dipropylenglykolmonomethylether;

Glycerin;

aromatische Alkohole wie Benzylalkohol;
Carbonsäureester wie z.B. Ethylacetat, Benzylbenzoat, Butylacetat, Propylencarbonat, Milchsäureethylester;
aliphatische Kohlenwasserstoffe wie Paraffine, Cyclohexan, Methylenchlorid, Ethylenchlorid;
aromatische Kohlenwasserstoffe wie Xylol, Toluol, Alkylnaphthaline, Chlorbenzole;
Ketone wie z.B. Aceton und Methylethylketon, Methylisobutylketon, Cyclohexanon;
natürliche und synthetische Mono- und Triglyceride mit natürlichen Fettsäuren wie Baumwollsaatöl, Erdnußöl, Maiskeimöl, Olivenöl, Ricinusöl, Sesamöl;
weiterhin Dimethylsulfoxid, Dimethylacetamid, Dimethylformamid, N-Methylpyrrolidon, Dioxan, 2,2-Dimethyl-4-oxymethyl-1,3-dioxolan.

Zu den oberflächenaktiven Stoffen zählen:
Emulgatoren und Netzmittel wie anionaktive Tenside, z.B. Alkylsulfonate, Alkylsulfate, Arylsulfonate, Na-Laurylsulfate, Fettalkoholethersulfate, Mono/Dialkylpolyglykoletherorthophosphorsäureester-Monoethanolaminsalz, Calciumalkylarylsulfonat;
kationaktive Tenside, z.B. Cetyltrimethylammoniumchlorid;
ampholytische Tenside, z.B. Di-Na-N-lauryl-beta-iminodipropionat oder Lecithin;
nichtionogene Tenside, z.B. polyoxyethyliertes Ricinusöl, polyoxyethyliertes Sorbitan-Monooleat, polyoxyethyliertes Sorbitan-Monostearat, Glycerinmonostearat, Polyoxyethylenstearat, Alkylphenolpolyglykolether, polyoxyethyliertes Sorbitanmonopalmitat, Polyoxyethylenlaurylether, Polyoxyethylen-oleylether, Polyoxyethylenmannitanmonolaurat, Alkylpolyglykolether, Oleylpolyglykolether, Dodecylpolyglykolether, ethoxyliertes Nonylphenol, Isooctylphenolpolyethoxyethanol.

Die Zubereitungen können außerdem enthalten:
Haftvermittler, z.B. Carboxymethylcellulose, Methylcellulose und andere Cellulose- und Stärke-Derivate, Polyacrylate, Alginate, Gelatine, Gummi arabicum, Polyvinylpyrrolidon, Polyvinylalkohol, Copolymere aus Methylvinylether und Maleinsäureanhydrid, Polyethylenglykole, Paraffine, Öle, Wachse, hydriertes Rizinusöl, Lecithine und synthetische Phospholipide.

Die Zubereitungen können Farbstoffe wie anorganische Pigmente, z.B.Eisenoxid, Titanoxid, Ferrocyanblau und organische Farbstoffe, wie Alizarin-, Azo- und Metallphthalocyaninfarbstoffe enthalten.

Die Zubereitungen können Spreitmittel enthalten, z.B. Silikonöle verschiedener Viskosität, Fettsäureester wie Ethylstearat, Di-n-butyl-adipat, Laurinsäurehexylester, Dipropylen-glykolpelargonat, Ester einer verzweigten Fettsäure mittlerer Kettenlänge mit gesättigten Fettalkoholen $C_{16}$-$C_{18}$, Isopropylmyristat, Isopropylpalmitat, Capryl/Caprinsäureester von gesättigten Fettalkoholen der Kettenlänge $C_{12}$-$C_{18}$, Isopropylstearat, Ölsäureoleylester, Ölsäuredecylester, Ethyloleat, Milchsäureethylester, wachsartige Fettsäureester, Dibutylphthalat, Adipinsäurediisopropylester, letzterem verwandte Estergemische u.a.;
Triglyceride wie Capryl/Caprinsäuretriglycerid, Triglyceridgemische mit Pflanzenfettsäuren der Kettenlänge $C_8$-$C_{12}$ oder anderen speziell ausgewählten natürlichen Fettsäuren, Partialglyceridgemische gesättigter oder ungesättigter evtl. auch hydroxylgruppenhaltige Fettsäuren, Monodiglyceride der $C_8$/$C_{10}$-Fettsäuren und andere;
Fettalkohole wie Isotridecylalkohol, 2-Octyldodecanol, Cetylstearylalkohol, Oleylalkohol.

Zur Herstellung von Pulvern und Stäuben wird der Wirkstoff mit geeigneten Trägerstoffen gegebenenfalls unter Zusatz von Hilfsstoffen vermischt und in die gewünschte Form gebracht.

Als Trägerstoffe seien genannt alle physiologisch verträglichen festen Inertstoffe. Als solche dienen anorganische und organische Stoffe. Anorganische Stoffe sind gegebenenfalls gebrochene und fraktionierte, z.B. synthetische und natürliche Gesteinsmehle wie Kaoline, Talkum, Kreide, Diatomeenerde, Kochsalz, Aluminiumoxide, Kieselsäuren, Tonerden, gefälltes oder kolloidales Siliciumdioxid. Organische Stoffe sind Laktose, Stärke und Cellulose bzw. Cellulosederivate.

Hilfsstoffe sind Konservierungsstoffe, Antioxidantien, Farbstoffe, die bereits weiter oben aufgeführt worden sind.

Weitere geeignete Hilfsstoffe sind Schmier- und Gleitmittel wie z.B. Magnesiumstearat, Stearinsäure, Talkum, Bentonite.

Die Wirkstoffe können in Form ihrer oben erwähnten festen oder flüssigen Formulierungen auch eingekapselt vorliegen.

Die Wirkstoffe können auch in Form eines Aerosols angewendet werden. Dazu wird der Wirkstoff in geeigneter Formulierung unter Druck fein verteilt.

Es kann auch vorteilhaft sein, die Wirkstoffe in Formulierungen anzuwenden, die den Wirkstoff verzögert freigeben. Als solche seien wirkstoffhaltige Formkörper wie z.B. Platten, Bänder, Streifen, Halsbänder, Gliedmaßenbänder, Markierungsvorrichtungen genannt.

Die Wirkstoffe können in den Formulierungen allein oder in Mischung mit anderen Wirkstoffen oder Synergisten vorliegen.

Direkt angewendete Formulierungen enthalten zwischen $10^{-7}$ und 5 Gewichtsprozent, bevorzugt zwischen $10^{-4}$ und 1 Gewichtsprozent Wirkstoff.

Formulierungen die erst nach weiterer Verdünnung angewendet werden enthalten 1 - 95 Gewichtsprozent, bevorzugt 5 - 90 Gewichtsprozent Wirkstoff.

Formulierungsbeispiele

| 1. Herstellung eines Emulsionskonzentrates | | |
|---|---|---|
| a) | Wirkstoff gemäß Beispiel 1 (100 %)<br>nichtionischer Emulgator (Emulgator 368® = Alkylarylpolyglycolether MG ca. 1165)<br>Dipropylenglykolmonomethylether ad | 25,00 g<br>25,00 g<br>100,00 ml |

Herstellung

Die Substanzen werden zusammen gewogen und so lange gerührt, bis eine klare Lösung entstanden ist.

Vor Gebrauch wird die Lösung auf ihre Anwendungskonzentration verdünnt.

| | | |
|---|---|---|
| b) | Wirkstoff gemäß Beispiel 2 (100 %)<br>Polyoxyethylenstearat<br>Sorbitan-Sesquioleat<br>Wasser<br>Polyethylenglykol 200 ad 100 ml | 1,00 g<br>0,50 g<br>0,40 g<br>4,00 g |

Herstellung und Anwendung wie bei 1a

| 2. Herstellung einer pour-on-Formulierung | | |
|---|---|---|
| a) | Zusammensetzung | |
| | Wirkstoff gemäß Beispiel 1 (100 %)<br>Isopropylmyristat<br>2-Octyldodecanol<br>Isopropanol ad 100 ml | 5,00 g<br>30,00 g<br>20,00 g |

Herstellung:

Die Substanzen werden zusammen gewogen und bis zur klaren Lösung gerührt.

| b) | Zusammensetzung | |
|---|---|---|
| | Wirkstoff gemäß Beispiel 1 (100 %)<br>Silikonöl 100<br>Butylacetat ad 100 ml | 0,50 g<br>30,00 g |

Herstellung wie bei Beispiel a)

| 3. Herstellung einer Mikroemulsion | |
|---|---|
| Wirkstoff gemäß Beispiel 1 (100 %) | 13,00 g |
| Eumulgin B3® (Alkylarylpolyglycolether) | 30,00 g |
| Cetiol HE® (Polyolfettsäureester) | 30,00 g |
| Isopropylmyristat | 5,00 g |
| Benzylalkohol | 3,00 g |
| Wasser ad 100 ml | |

Herstellung:

Der Wirkstoff wird in den lipophilen Komponenten (Eumulgin, Cetiol, Benzylalkohol, Isopropylmyristat) dispergiert.

Nach Erwärmen auf 60° C wird Wasser der gleichen Temperatur zugemischt und abgekühlt. Die entstandene Mikroemulsion gleicht außerlich einer klaren Lösung.

4. Herstellung einer Sprühformulierung

| Zusammensetzung | |
|---|---|
| Wirkstoff gemäß Beispiel 1 (100 %) | 20 g |
| Emulgator Toximul®3 (Mischung aus Alkylbenzolsulfonat-Ca und nichtionogenen Emulgatoren und Methanol) | 7 g |
| Emulgator Toximul S® (Mischung aus Alkylbenzolsulfonat-Ca und nichtionogenem Emulgator und Methanol) | 5 g |
| Sovesso 200® (Alkylnaphthalin-Gemisch hochsiedender Erdölfraktionen) | ad 100 ml |

Herstellung:

Der Wirkstoff wird mit den restlichen Komponenten zusammen gewogen, gerührt und vor Anwendung mit Wasser auf die Anwendungskonzentration verdünnt.

<u>Anwendungsbeispiele</u>

A. In vitro-Test an Flöhen (alle Entwicklungsstadien)

Testobjekt: Alle Stadien (Eier, Larven, Puppen, Adulte) von Ctenocephalides felis

Testverfahren

Die zu testende Substanz wird in der gewünschten Konzentration homogen in Blutmehl verteilt. In dieses Gemisch aus Blutmehl und Testsubstanz werden Floheier, die von flohbefallenen Katzen gesammelt wurden, gebracht und bei 80 % rel. Feuchte und 25° C inkubiert.

Nach Ablauf der Entwicklungszeit von 3 bis 4 Wochen wird festgestellt, ob adulte Flöhe zur Entwicklung gekommen sind. Dabei bedeutet eine 100 %ige Wirkung, daß keine adulten Flöhe festgestellt wurden und 0

%, daß lebende adulte Flöhe festgestellt wurden.

Der Wirkstoff gemäß Beispiel 1 zeigt bei 5 ppm 100 % Wirkung.

B. In vivo-Test an Flöhen (alle Entwicklungsstadien)

Testobjekt: Eier, Larven, Puppen von Ctenocephalides felis in der Umgebung von Hunden/Katzen, adulte Flöhe auf Hunden/Katzen.

Testverfahren

Läger von flohfreien Hunden/Katzen in Boxen werden mit der zu testenden Substanz in der gewünschten Anwendungskonzentration besprüht. Zu bestimmten Zeiten nach der Applikation werden Eier von Ctenocephalides felis, die von behandelten Katzen gesammelt wurden, auf die Läger der Hunde/Katzen gebracht. Es wird festgestellt, ob und zu welchem Zeitpunkt die in den Boxen befindlichen Hunde/Katzen von adulten Flöhen befallen werden.

Dabei bedeutet eine 100 %ige Wirkung, daß Hunde/Katzen keinen Flohbefall aufweisen und 0 % Wirkung, daß Hunde/Kazen von Flöhen befallen sind.

Der Wirkstoff gemäß Beispiel 1 zeigt 100 % Wirkung.

Herstellungsbeispiele

Verfahren 2a:

Allgemeine Vorschrift:

Ein Gemisch aus 1 m Verbindung der Formel II und der etwa 1,1- bis 3-fachen molaren Menge Verbindung der Formel III, der etwa 6-fachen molaren Menge Na-OH-Pulver, der etwa 1,1-fachen Menge $K_2CO_3$-Pulver sowie der etwa 0,02-fachen Menge des Phasentransferkatalysators Tris-(2-(2-methoxyethoxy)-ethyl)-amin wird in Toluol ca. 36 Stunden am Rückfluß gekocht. Nach dem Abkühlen wird die Toluolphase mit Wasser gewaschen, über $Na_2SO_4$ getrocknet und in Vakuum eingeengt. Das entstandene Produkt wird durch Kugelrohrdestillation weiter gereinigt. Gemäß dieser Vorschrift werden erhalten:

Beispiel 1

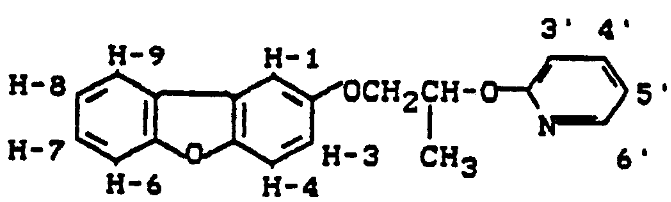

$^1$H-NMR (DMSO-$d_6$):

8,20 ppm (m, H-6′); 8,13 ppm (m, H-9); 7,80 ppm (m, H-1); 7,72 ppm (m, H-4′); 7,67 ppm (m, H-4); 7,60 ppm (m, H-6); 7,51 ppm (m, H-7); 7,39 ppm (m, H-8); 7,13 ppm (m, H-3); 6,99 ppm (m, H-5′); 6,84 ppm (m, H-3′); 5,60 ppm (m, -CH-O); 4,28 ppm (m, 2H, $-OCH_2$); 1,45 ppm (d, J = 6,4 Hz; $CH_3$).

Das Salz mit HCl hat einen Fp von 109° C.

Beispiel 2

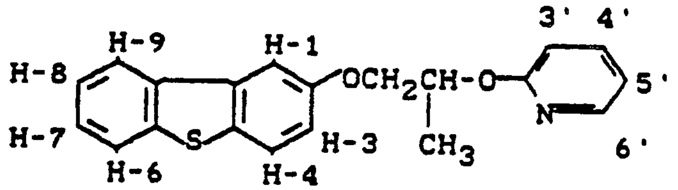

$^1$H-NMR, $CDCl_3$:
8,18 ppm (m, H-6'); 8,08 ppm (m, H-9); 7,81 ppm (m, H-4'); 7,70 ppm (m, H-4); 7,68 ppm (m, H-1); 7,58-7,4 ppm (m, 2H, H-7, H-6); 7,1 ppm (m, H-3); 6,85 ppm (m, H-5'); 6,76 ppm (m, H-3'); 5,65 ppm (m, CH); 4,33 ppm + 4,20 ppm (m, 2H, O$\underline{CH_2}$); 1,51 ppm (d, $CH_3$).

Beispiel 3

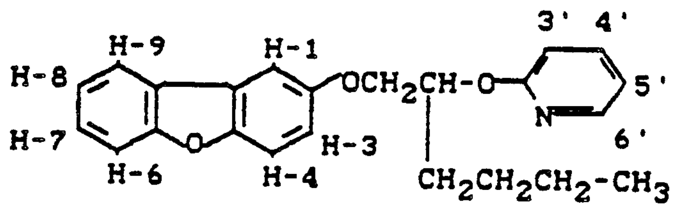

$^1$H-NMR, $CDCl_3$:
8,16 ppm (m, H-6'); 7,85 ppm (m, H-9); 7,55 ppm (m, H-4'); 7,50 ppm (m, H-4); 7,48 ppm (m, H-1); 7,4 ppm (m, 2H, H-7, H-6); 7,29 ppm (m, H-8); 7,05 ppm (m, H-3); 6,83 ppm (m, H-5'); 6,76 ppm (m, H-3'); 5,57 ppm (m, 1H, CH); 4,27 ppm (m, 2H, O$\underline{CH_2}$); 1,91-1,45 ppm (m, 6H, Butyl $CH_2$); 0,91 ppm (t, 3H, $CH_3$).

Beispiel 4

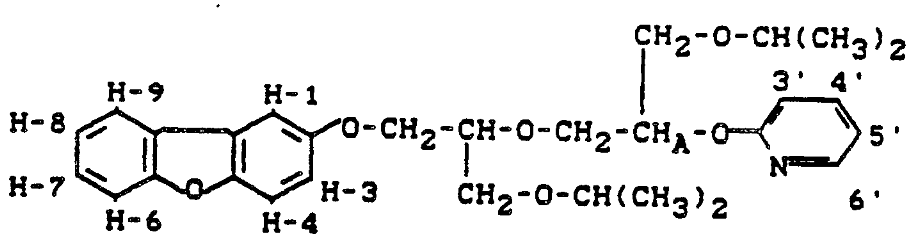

$^1$H-NMR, $CDCl_3$:
8,10 ppm (m, H-6'); 7,88 ppm (m, H-9); 7,52 ppm (m, H-4); 7,50 ppm (m, H-4'); 7,45-7,40 ppm (m, 3H, H-1, H-6, H-7); 7,40 ppm (m, H-8); 7,03 ppm (m, H-3); 6,78 ppm (m, H-5'); 6,74 ppm (m, H-3'); 5,45 ppm (m, 1H, $CH_A$); 4,2-3,5 ppm (m, 11H); 1,10 ppm (m, 12H, $CH_3$).

Beispiel 5

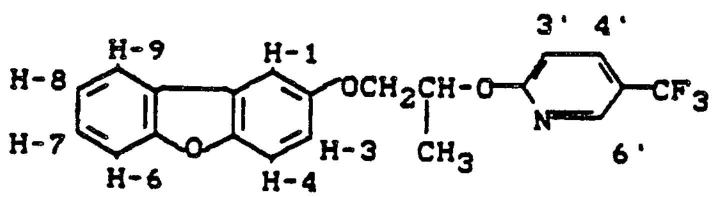

$^1$H-NMR, $CDCl_3$:

8,45 ppm (s, H-6′); 7,85 ppm (m, H-9); 7,74 ppm (d, H-4′); 7,51 ppm (m, H-4); 7,45 ppm (m, 3H; H-1, H-6, H-7); 7,3 ppm (m, H-8); 7,05 ppm (m, H-3); 6,80 ppm (d, H-3′); 5,69 ppm (m, CH); 4,28 ppm + 4,18 ppm (m, 2H, $OCH_2$); 1,52 ppm (d, $CH_3$).

Beispiel 6

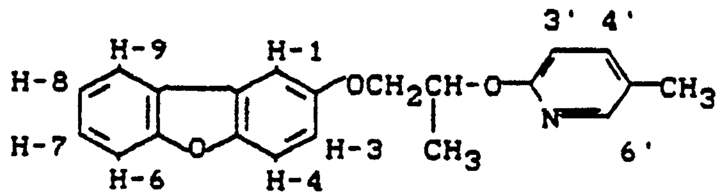

$^1$H-NMR, $CDCl_3$:

7,97 ppm (s, H-6′); 7,88 ppm (m, H-9); 7,52 ppm (m, H-4); 7,47 ppm (m, H-1); 7,40 ppm (m, 2H, H-7, H-6); 7,39 ppm (d, H-4′); 7,30 ppm (m, H-8); 7,08 ppm (m, H-3); 6,68 ppm (d, H-3′); 5,59 ppm (m, CH); 4,29 ppm + 4,16ppm (m, 2H, $OCH_2$); 2,22 ppm (s, $CH_3$ Pyridin); 1,51 ppm (d, J = 6,4 Hz, $CH_3$).

Beispiel 7

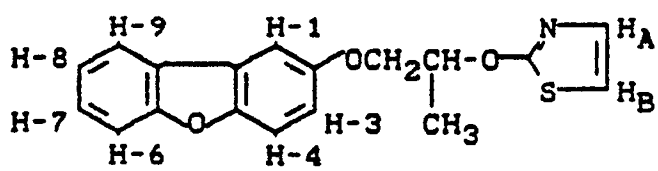

$^1$H-NMR, $CDCl_3$:

7,87 ppm (m, H-9); 7,53 ppm (m, H-4); 7,41 ppm (m, 3H, H-1, H-6, H-7); 7,30 ppm (m, H-8); 7,14 ppm (d, J = 3,8 Hz, $H_A$ oder $H_B$); 7,05 ppm (m, H-3); 6,68 ppm (d, J = 3,8 Hz, $H_B$ oder $H_A$); 5,50 ppm (m, CH); 4,26 ppm (m, $CH_2$, 2H); 1,58 ppm (d, J = 6,4 Hz, $CH_3$).

Beispiel 8

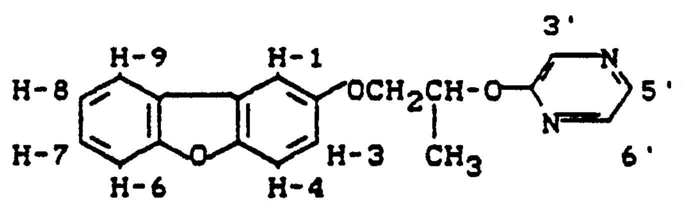

$^1$H-NMR, $CDCl_3$:

8,25 ppm (d, J = 1,3 Hz, H-3′); 8,12 ppm (d, J = 2,8 Hz, H-6′); 8,07 ppm (dd, H-5′); 7,89 ppm (m, H-9); 7,54 ppm (m, H-4); 7,48 ppm (m, H-1); 7,45 ppm (m, 2H, H-6), H-7); 7,30 ppm (m, H-8); 7,05 ppm (m, H-3); 5,61 ppm (m, CH); 4,30 ppm und 4,19 ppm (m, 2H, $CH_2$); 1,53 ppm (d, J = 6,4 Hz, $CH_3$).

Beispiel 9

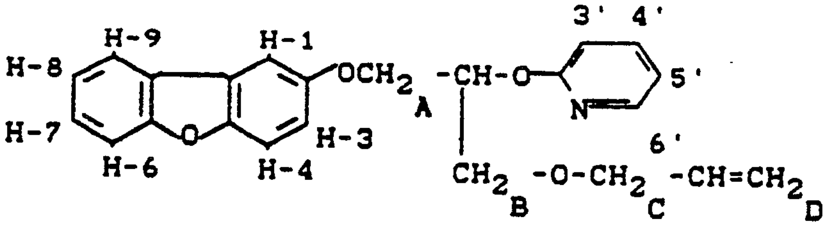

$^1$H-NMR, $CDCl_3$:
8,12 ppm (m, H-6′); 7,85 ppm (m, H-9); 7,52 ppm (m, H-4′); 7,5-7,4 ppm (m, 4H; H-1), H-4, H-6, H-7); 7,25 ppm (m, H-8); 7.08 ppm (m, H-3); 6,82 ppm (m, H-5′); 6,79 ppm (m, H-3′); 5,89 ppm (m, 1H, CH = $CH_2$); 5,72 ppm (m, -CH-O); 5,28 + 5,18 ppm (m, 2H, CH = $CH_2$); 4,39-3,90 ppm (m, 6H, $CH_{2A}$ + $CH_{2B}$ + $CH_{2C}$).

Beispiel 10

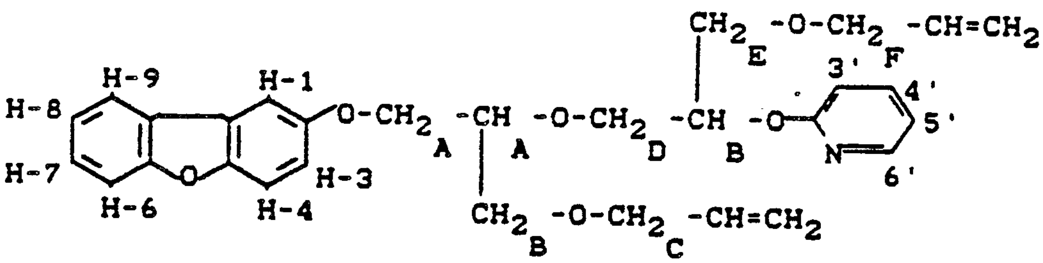

$^1$H-NMR, $CDCl_3$:
8.09 ppm (m, H-6′); 7,85 ppm (m, H-9); 7,50 ppm (m, H-4); 7,48 ppm (m, H-4′); 7,38 ppm (m, 3H, H-1, H-6, H-7); 7,28 ppm (m, H-8); 7,00 ppm (m, H-3); 6,78 ppm (m, H-5′); 6,73 ppm (m, H-3′); 5,88 ppm (m, 2H, -CH = $CH_2$); 5,80-5,60 ppm (m, 4H, CH = $CH_2$); 5,52 ppm (m, 1H, $CH_B$); 4,2-3,6 ppm (m, 13H), $CH_{2A-F}$, $CH_A$).

Beispiel 11

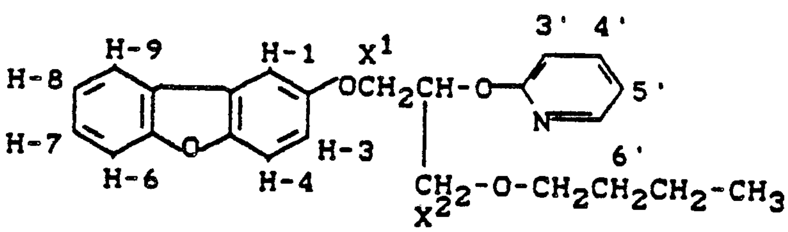

Schmelzpunkt: 44 °C
$^1$H-NMR, $CDCl_3$:
8,15 ppm (m, H-6′); 7,85 ppm (m, H-9); 7,54 ppm (m, H-4′); 7,50 ppm (m, H-4); 7,48 ppm (m, H-1); 7,4 ppm (m, 2H, H-6, H-7); 7,29 ppm (m, H-8); 7,08 ppm (m, H-3); 6,85 ppm (m, H-5′); 6,80 ppm (m, H-3′); 5,7 ppm (m, -CH-O); 4,38 ppm + 3,87 ppm (d, 4H, $CH_2X_1$, $CH_2X_2$); 3,52 ppm (t, 2H, $-OCH_2(CH_2)_2CH_3$); 1,57 ppm (m, 2H, $-OCH_2-CH_2-CH_2CH_3$); 1,32 ppm (m, 2H, $-OCH_2CH_2CH_2CH_3$); 0,88 ppm (t, 3H, $-O-(CH_2)_3-CH_3$).

Beispiel 12

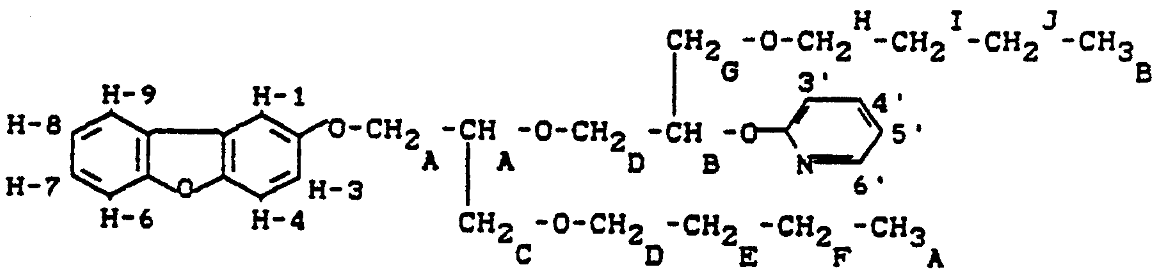

$^1$H-NMR, $CDCl_3$:
8,11 ppm (m, H-6'); 7,86 ppm (m, H-9); 7,53 ppm (m, H-4); 7,52 ppm (m, H-4'); 7,45 ppm (m, 3H, H-1, H-6, H-7); 7,29 ppm (m, H-8); 7,02 ppm (m, H-3); 6,79 ppm (m, H-5'); 6,74 ppm (m, H-3'); 5,5 ppm (m, $CH_A$); 4,15 ppm (m, 2H, $CH_{2A}$); 4,00 ppm (m, 3H, $CH_{2B}$ + $CH_B$); 3,75 ppm + 3,62 ppm (m, 4H, $CH_{2C}$ + $CH_{2G}$); 3,48 ppm (m, 4H, $CH_{2D}$ + $CH_{2H}$); 1,52 ppm (m, 4H, $CH_{2E}$ + $CH_{2I}$); 1,35 ppm (m, 4H, $CH_{2F}$ + $CH_{2J}$); 0,88 ppm + 0,87 ppm (t, 6H, $CH_{3A}$ + $CH_{3B}$).

Beispiel 13

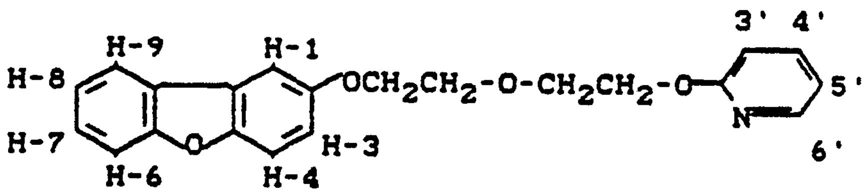

$^1$H-NMR (DMSO-$d_6$):
8,17-8,15 ppm (m, H-6'); 8,13-8,10 ppm (m, H-9); 7,75 ppm (m, H-1); 7,72 ppm (m, H-4'); 7,67 ppm (m, H-4); 7,59 ppm (m, H-6); 7,51 ppm (m, H-7); 7,38 ppm (m, H-8); 7,09 ppm (m, H-3); 6,97 ppm (m, H-5'); 6,83 ppm (m, H-3'); 4,43-4,40 ppm (m, 2H); 4,24-4,21 ppm (m, 2H); 3,88-3,84 ppm (m, 4H).

Beispiel 14

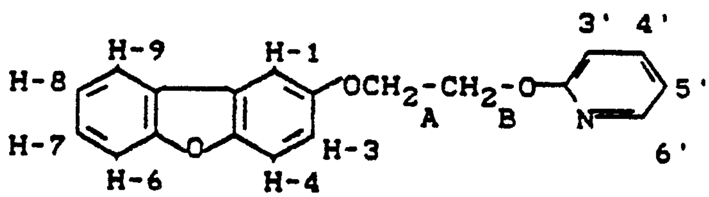

$^1$H-NMR, $CDCl_3$:
8,17 ppm (m, H-6'); 7,89 ppm (m, H-9); 7,56 ppm (m, H-4'); 7,55-7,41 ppm (m, 4H, H-1, H-4, H-6, H-7); 7,31 ppm (m, H-8); 7,10 ppm (m, H-3); 6,90 ppm (m, H-5'); 6,83 ppm (m, H-3'); 4,74 ppm (t, J = 4,9 Hz, $CH_{2B}$); 4,43 ppm (t, $CH_{2A}$).

Beispiel 15

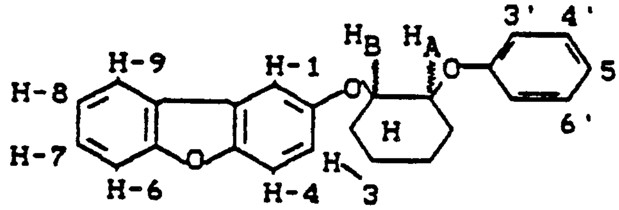

$^1$H-NMR, DMSO-$d_6$:
8,18 ppm (m, H-6'); 8,12 ppm (m, H-9); 7,80 ppm (m, H-1); 7,65 ppm (m, 2H, H-4 und H-4'); 7,55 ppm (m, H-6); 7,51 ppm (m, H-7); 7,38 ppm (m, H-8); 7,08 ppm (m, H-3); 6,95 ppm (m, H-5'); 6,68 ppm (m, H-3'); 5,32-5,27 ppm (m, $H_A$); 4,61-4,54 ppm (m, $H_B$); 2,20-1,46 ppm (m, 8H).

Beispiel 16

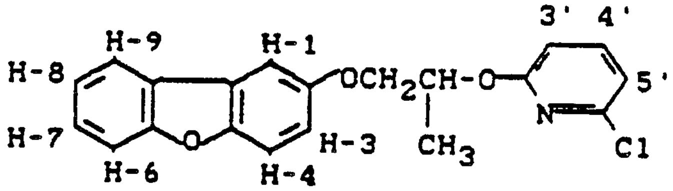

$^1$H-NMR, DMSO-$d_6$:
8,14 ppm (m, H-9); 7,77 ppm (m, 2H, H-1 + H-4'); 7,66 ppm (m, H-4); 7,60 ppm (m, H-6); 7,51 ppm (m, H-7); 7,38 ppm (m, H-8); 7,11 ppm (m, 2H, H-3 + H-5'); 5,50 ppm (m, CH); 4,27 ppm (m, $OCH_2$); 1,44 ppm (d, J = 6,4 Hz, $CH_3$).

Beispiel 17

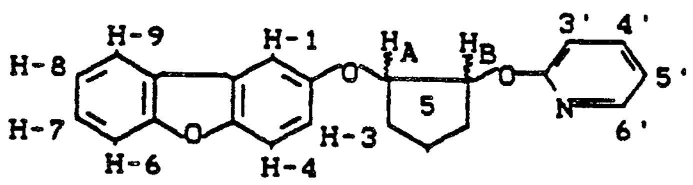

$^1$H-NMR, DMSO-$d_6$:
8,20-8,18 ppm (m, H-6'); 8,10-8,07 ppm (m, H-9); 7,84-7,83 ppm (m, H-1); 7,73 ppm (m, H-4'); 7,66 ppm (m, H-4); 7,62-7,59 ppm (m, H-6); 7,51 ppm (m, H-7); 7,38 ppm (m, H-8); 7,19-7,16 ppm (m, H-3); 7,02-6,97 ppm (m, H-5'); 6,88-6,85 ppm (m, H-3'); 5,48-5,46 ppm (m, $H_B$); 4,97-4,95 ppm (m, $H_A$); 2,27-1,84 ppm (m, 6H).

Beispiel 18

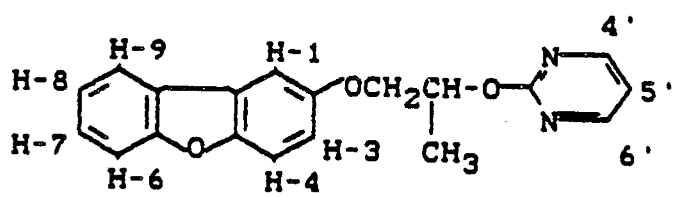

$^1$H-NMR, DMSO-$d_6$:
8,64-8,62 ppm (m, 2H, H-4′ + H-6′); 8,15-8,12 ppm (m, H-9); 7,8-7,79 ppm (m, H-1); 7,68-7,65 ppm (m, H-4); 7,62-7,59 ppm (m, H-6); 7,51 ppm (m, H-7); 7,39 ppm (m, H-8); 7,15-7,10 ppm (m, 2H, H-5′ + H-3).

Beispiel 19

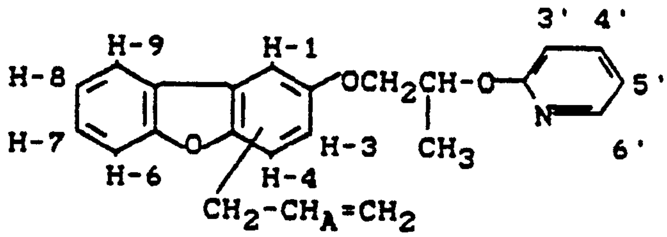

$^1$H-NMR (DMSO-$d_6$):
2 Hauptisomere i.V. ~ 3:1
8,20-8,18 ppm (m, H-6′); 8,18-8,12 ppm (m, H-9); 7,75-7,72 ppm (m, H-4′); 7,75 ppm (m, 1H); 7,69-7,65 ppm (m, 1H); 7,53-7,48 ppm (m, 2H, H-6, H-7); 7,38-7,33 ppm (m, H-8); 7,26-7,21 ppm (m, 1H); 7,00-6,96 ppm (m, H-5′); 6,83-6,77 ppm (m, H-3′); 6,78 ppm (m, $CH_A = CH_2$); 6,55-6,50 ppm (m, 2H, $CH_A = CH_2$); 5,65 ppm (m, $-CH-CH_3$, 1H); 4,2 ppm (m, $OCH_2$); 2,06-1,85 ppm (m, $-CH_2-CH=CH_2$); 1,42 ppm (d, J = 6,4 Hz, $CH_3$ Hauptisomeres); 1,22 ppm (d, J = 6,2 Hz, $CH_3$ Nebenisomeres).

Beispiel 20

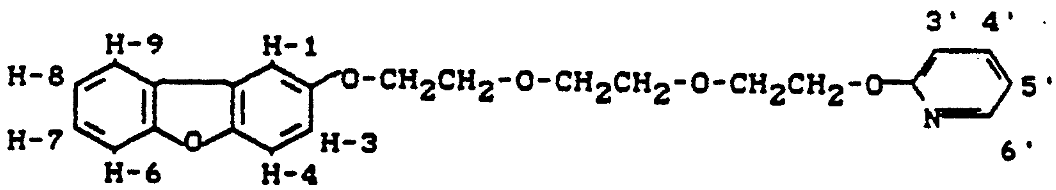

$^1$H-NMR, DMSO-$d_6$:
8,15-8,10 ppm (m, 2H, H-9, H-6′); 7,74 ppm (m, H-1); 7,71-7,65 ppm (m, 2H, H-4, H-4′); 7,61-7,58 ppm (m, H-6); 7,53-7,48 ppm (m, H-7); 7,4-7,35 ppm (m, H-8); 7,13-7,09 ppm (m, H-3); 7,00-6,94 ppm (m, H-5′); 6,83-6,8 ppm (m, H-3′); 4,38-4,35 ppm (m, 2H); 4,21-4,18 ppm (m, 2H); 3,82-3,74 ppm (m, 4H); 3,65-3,62 ppm (m, 4H).

Verfahren 2b:

Allgemeine Vorschrift:

Ein Gemisch aus ca. 20 mmol Verbindung der Formel IV und der etwa 1,5 - bis 2-fachen molaren Menge Verbindung der Formel V, der etwa 0,05-fachen Menge des Phasentransferkatalysators (Adogen 464) wird in 30 ml 45 %iger NaOH und 30 ml Methylenchlorid 18 Stunden bei 50 bis 70 °C gerührt. Nach dem Abkühlen wird mit 0,5 l Wasser verdünnt, dreimal mit jeweils 50 ml Methylenchlorid extrahiert, die Methylenchloridphasen eingeengt und der verbleibende Rückstand aus Diethylether umkristallisiert. Gemäß dieser Vorschrift wird erhalten:

Beispiel 21

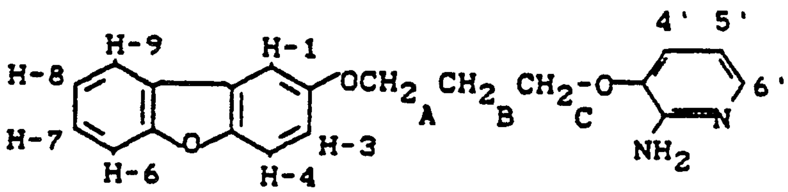

$^1$H-NMR, (DMSO-$d_6$):
8,11 ppm (m, H-9); 7,75 ppm (m, H-1); 7,68 ppm (m, H-4); 7,60 ppm (m, H-6); 7,50 ppm (m, H-7, H-6′); 7,37 ppm (m, H-8); 7,12 ppm (m, H-3); 7,02 ppm (m, H-4′); 6,5 ppm (m, H-5′); 4,30 ppm und 4,12 ppm (t, $CH_{2A}$ und $CH_{2C}$); 2,25 ppm (m, $CH_{2B}$).

Verfahren 2c:

Allgemeine Vorschrift:

9,4 mmol Verbindung der Formel I werden mit 29,6 mmol Sulfurylchlorid in 100 ml trockenem Toluol gerührt. Nach 18 Stunden wird das Lösungsmittel abdestilliert und der Rückstand chromatographisch an Kieselgel mit Methylenchlorid/Cyclohexan = 1:1 als Laufmittel gereinigt.

Gemäß diesem Verfahren wird erhalten:

Beispiel 22

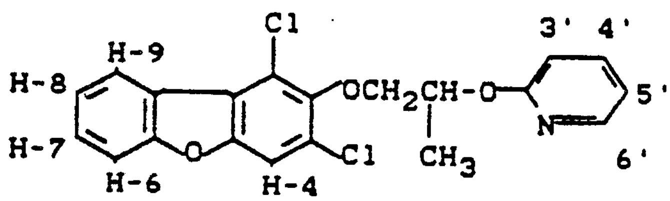

$^1$H-NMR, DMSO-$d_6$:
8,29 ppm (m, H-9); 8,17 ppm (m, H-6′); 8,00 ppm (s, H-4); 7,76 ppm (m, H-6); 7,68 ppm (m, H-4′); 7,65 ppm (m, H-7); 7,48 ppm (m, H-8); 6,98 ppm (m, H-5′); 6,78 ppm (m, H-3′); 5,59 ppm (m, CH); 4,25 ppm (m, $CH_2$); 1,48 ppm (d, J = 6,6 Hz, $CH_3$).

Beispiel 23

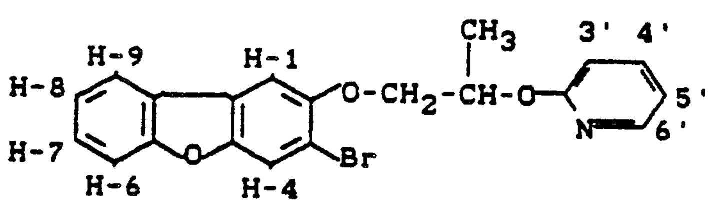

1,3 g (4,1 mmol) substituiertes Dibenzofuran der Formel I wird mit 1,6 g (10 mmol) Brom in 70 ml Eisessig 24 Stunden bei Raumtemperatur gerührt. Anschließend wird der entstandene orangerote Niederschlag abfiltriert, mit Wasser gewaschen und getrocknet.
Ausbeute: 80 %
$^1$H-NMR, DMSO-$d_6$:
8,20 ppm (m, H-6′); 8,15 ppm (m, H-9); 8,00 ppm (s,H-4); 7,99 ppm (S, H-1); 7,82 ppm (m,H-4′); 7,69 ppm (m, H-6); 7,55 ppm (m, H-7); 7,40 ppm (m, H-8); 7,05 ppm (m, H-5′); 6,99 ppm (m, H-3′); 5,61 ppm (m,

m (m, $CH_2$); 1,50 ppm (d,J=7Hz, $CH_3$).

ler Ausgangsverbindungen der Formel II gemäß Verfahren 4:

Verbindung der Formel VI werden 3 bis 5 mol Epoxid der Formel VII in Gegenwart von 2 bis 3 ),005 bis 0,02 mol Phasentransferkatalysator Tetrabutylammoniumbromid in einer Mischung aus ıd ca. 340 ml Wasser bei 50 bis 130 °C 48 Stunden gerührt. Nach dem Abkühlen wird das t gesättigter NaCl-Lösung versetzt, und die Toluolphase abgetrennt. Toluol wird im Vakuum ınd der Rückstand destilliert.
lieser Vorschrift werden erhalten:

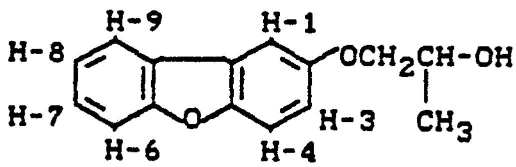

ИSO-$d_6$):
, H-9); 7,73 ppm (d, J = 2,6 Hz, H-1); 7,66 ppm (m, H-4); 7,59 ppm (m, H-6); 7,50 ppm (m, H-
(m, H-8); 7,10 ppm (m, H-3); 5,0 ppm (s, breit, OH); 4,01 ppm (m,

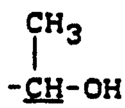 );

. OCH2); 1,21 ppm (d, J = 6,1 Hz; $CH_3$).

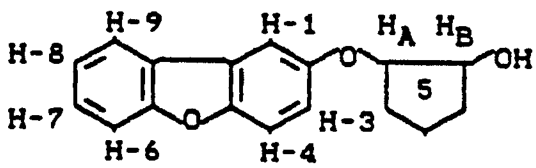

ISO-$d_6$):
, H-9); 7,73 ppm (m, H-1); 7,66 ppm (m, H-4); 7,59 ppm (m, H-6); 7,51 ppm (m, H-7); 7,38 ppm
) ppm (m, H-3); 4,59-4,57 ppm und 4,15 ppm (m, $H_A$ + $H_B$); 2,21-1,24 ppm (m, 6H, Ring).

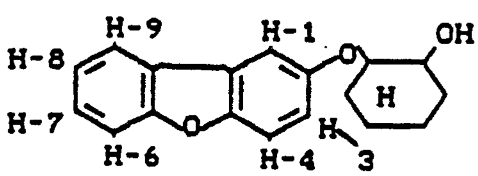

SO-$d_6$:

8,15 ppm (m, H-9); 7,80 ppm (m, H-1); 7,66 ppm (m, H-4); 7,58 ppm (m, H-6); 7,50 ppm (m, H-7); 7,38 ppm (m, H-8); 7,14 ppm (m, H-3);
Cycloalkyl: 4,11 ppm (m, 1H; 3,58 ppm (m, 1H); 2,09 ppm (m, 1H); 1,89-1,32 ppm (m, 7H).

Beispiel d)

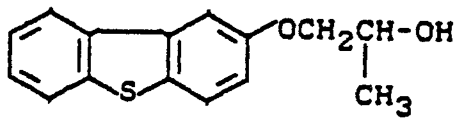

Ausbeute: 12,5 g (65 % d.Th.) hellbraunes Öl
Siedepunkt: 250° C/0,6 mm

Beispiel e)

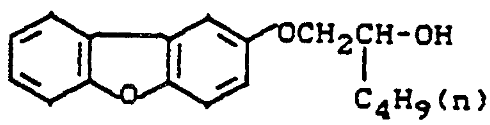

Ausbeute: 2,3 g (8 % d.Th.) gelbes Öl

Beispiel f)

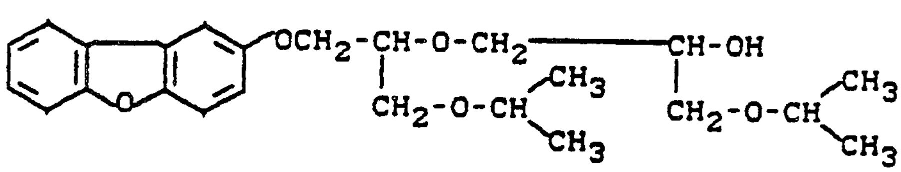

Ausbeute: 55,6 g (61 % d.Th.) gelbes Öl

Beispiel g)

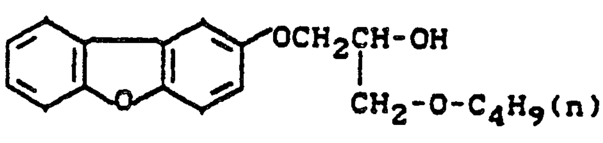

+

**3**

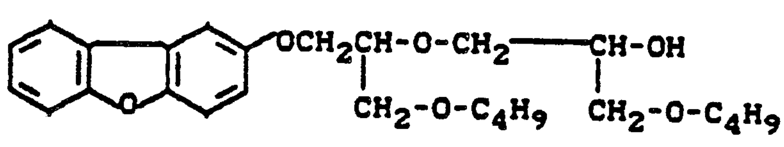

**4**

Ausbeute: 76,3 g [davon 40 % 3 und 60 % 4] hellbraunes Öl

Beispiel h)

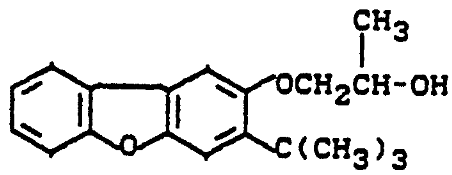

Ausbeute: 18,5 g (49 % d.Th.)

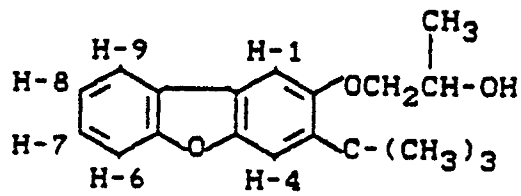

$^1$H-NMR, DMSO-$d_6$:

8,11 ppm (m, H-9); 7,72 ppm (s, H-1); 7,64 ppm (m, H-6); 7,52 ppm (s, H-4); 7,46 ppm (m, H-7); 7,36 ppm (m, H-8); 4,12 ppm (m, 1H, CH-$CH_3$); 4,10-3,94 ppm (m, 2H, $OCH_2$); 1,46 ppm (s, 9H, tert.-Butyl); 1,31 ppm (d, 3H, -CH-$CH_3$, J = 6,2 Hz).

Beispiel i)

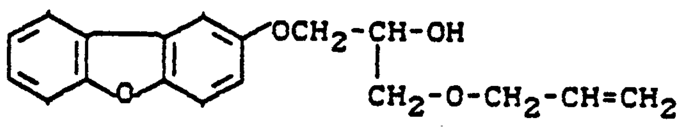

3

\+

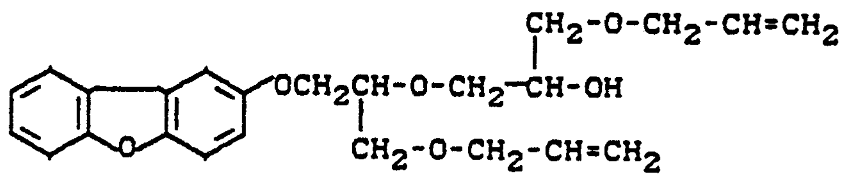

4

Ausbeute: 28,6 g [davon 56 % 3 und 44 % 4] hellbraunes Öl

Beispiel k)

15 g 2-Hydroxydibenzofuran wird mit 38 g 2-Bromethanol in Gegenwart von 50 g $K_2CO_3$ und 6 g KJ in 400 ml Acetonitril ca. 24 Stunden am Rückfluß erhitzt. Nach dem Abkühlen wird vom Lösungsmittel abdestilliert, der verbleibende Rückstand mit Natronlauge und Toluol gewaschen und anschließend die Toluolphase über $Na_2SO_4$ getrocknet. Es wird erneut vom Lösungsmittel abdestilliert. Man erhält gemäß dieser Vorschrift:

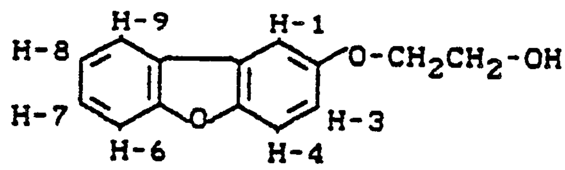

$^1$H-NMR, (DMSO-$d_6$):
8,15 ppm (m, H-9); 7,75 ppm (m, H-1); 7,68 ppm (m, H-4); 7,62 ppm (m, H-6); 7,52 ppm (m, H-7); 7,39 ppm (m, H-8); 7,13 ppm (m, H-3); 5,35 ppm (s, breit, OH); 4,13 ppm und 3,82 ppm (t, J = 5,0 Hz, -O-$CH_2CH_2$-O, 4H).

Beispiel l)

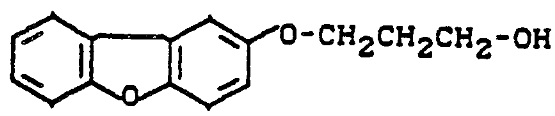

Ausbeute: 10,0 g (76 % d.Th.) braunes Öl
Siedepunkt: 230-250 ° C/0,5 mm

Beispiel m)

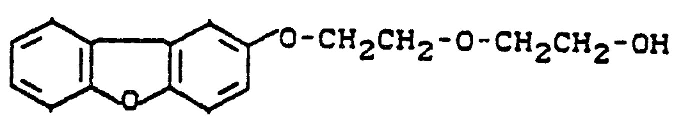

Ausbeute: 13,9 g (67 % d.Th.)
Siedepunkt: 230-250 ° C/0,5 mm

Beispiel n)

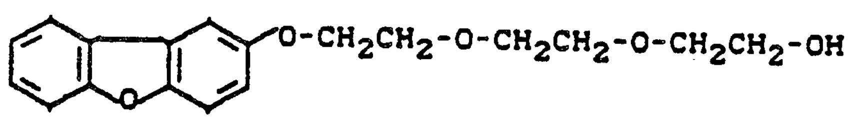

Ausbeute: 11,8 g (57 % d.Th.)
Siedepunkt: 250 ° C/0,4 mm

## Ansprüche

1. Substituierte tricyclische Verbindungen der Formel I

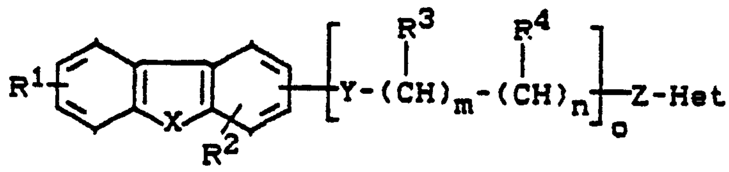

I

in welcher
X für O, S, NH, $-CH_2-$, $-CHCH_3$, $-C(CH_3)_2$-steht,
Y für O oder S steht,
Z für O oder S steht,
$R^1$ für gleiche oder verschiedene Reste aus der Gruppe Wasserstoff, Halogen, Alkyl, Alkoxy, Halogenalkyl steht,
$R^2$ für gleiche oder verschiedene Reste aus der Gruppe Wasserstoff, Halogen, Alkyl, Alkenyl, Halogenalkyl, Alkoxy, Alkylthio, Halogenalkoxy, Halogenalkylthio, steht,
$R^3$ für Wasserstoff, Alkyl, Halogenalkyl, Alkoxyalkyl, Alkenoxyalkyl, Alkenyl, Alkinyl steht,
$R^4$ für die bei $R^3$ angegebenen Reste steht,
$R^3$ und $R^4$ gemeinsam mit den angrenzenden C-Atomen für gesättigte oder ungesättigte 5- bis 7-gliedrige carbocyclische Ringe stehen können,
m für 1 oder 2 steht,
n für 1 oder 2 steht,
o für 1, 2 oder 3 steht und
Het für gegebenenfalls substituiertes Heteroaryl steht, das nicht über das Heteroatom an den übrigen Rest gebunden ist.

2. Verfahren zur Herstellung der substituierten tricyclischen Verbindungen der Formel I

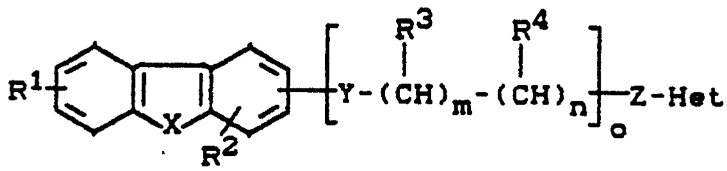

I

in welcher
X für O, S, NH, $-CH_2-$, $-CHCH_3-$, $-C(CH_3)_2$-steht,
Y für O oder S steht,
Z für O oder S steht,
$R^1$ für gleiche oder verschiedene Reste aus der Gruppe Wasserstoff, Halogen, Alkyl, Alkoxy, Halogenalkyl steht,
$R^2$ für gleiche oder verschiedene Reste aus der Gruppe Wasserstoff, Halogen, Alkyl, Alkenyl, Halogenalkyl, Alkoxy, Alkylthio, Halogenalkoxy, Halogenalkylthio, steht,
$R^3$ für Wasserstoff, Alkyl, Halogenalkyl, Alkoxyalkyl, Alkenoxyalkyl, Alkenyl, Alkinyl steht,
$R^4$ für die bei $R^3$ angegebenen Reste steht,
$R^3$ und $R^4$ gemeinsam mit den angrenzenden C-Atomen für gesättigte oder ungesättigte 5- bis 7-gliedrige carbocyclische Ringe stehen können,
m für 1 oder 2 steht,
n für 1 oder 2 steht,
o für 1, 2 oder 3 steht und
Het für gegebenenfalls substituiertes Heteroaryl steht, das nicht über das Heteroatom an den übrigen Rest gebunden ist,
die dadurch gekennzeichnet sind, daß man
a) Verbindungen der Formel II

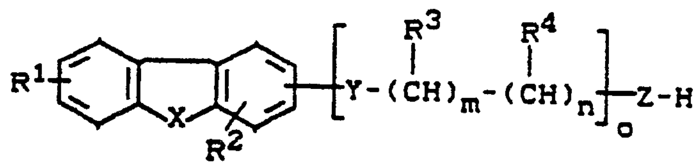

II

in welcher
$R^1$, $R^2$, $R^3$, $R^4$, X, Y, Z, m, n, o die oben angegebene Bedeutung haben,
mit Halogenheterocyclen der Formel III
Hal-Het III
in welcher
Hal für Halogen steht und
Het die oben angegebene Bedeutung hat,
umsetzt, oder

b) Verbindungen der Formel IV

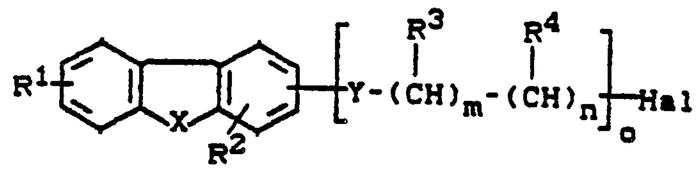

IV

in welcher
$R^1$, $R^2$, $R^3$, $R^4$, X, Y, m, n, o die oben angegebene Bedeutung haben, und
Hal für Halogen steht,
mit Hydroxy- oder Thioheterocyclen der Formel V
H-O(S)-Het V
in welcher
Het die oben angegebene Bedeutung hat,
umsetzt, oder

c) für den Fall, daß in Formel I $R^2$ für Halogen steht, man Verbindungen der Formel I, in welcher $R^2$ für Wasserstoff steht und die übrigen Substituenten die oben angegebenen Bedeutungen haben, mit Halogenierungsmitteln umsetzt.

3. Verbindungen der Formel II

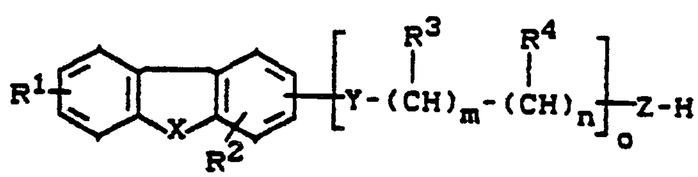

II

in welcher
$R^1$, $R^2$, $R^3$, $R^4$, X, Y, Z, m, n, o die oben angegebene Bedeutung haben.

4. Verfahren zur Herstellung der Verbindungen der Formel II, dadurch gekennzeichnet, daß man Verbindungen der Formel VI

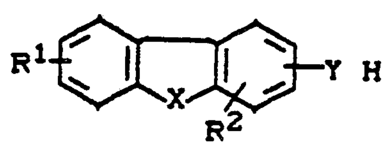

VI

in welcher
$R^1$, $R^2$, X, Y die oben angegebene Bedeutung haben,
mit Verbindungen der Formeln VII oder VIII

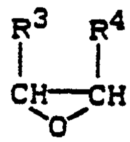

VII

in welcher
$R^3$ und $R^4$ die oben angegebene Bedeutung haben,

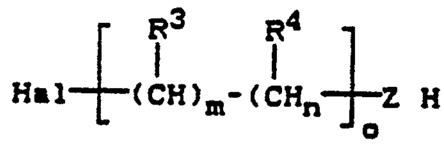

VIII

in welcher
Hal, $R^3$, $R^4$, Z, m, n, o die oben angegebene Bedeutung haben,
umsetzt.

5. Verbindungen der Formel IV

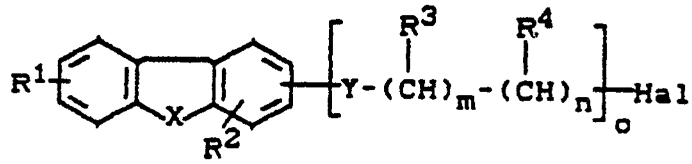

IV

in welcher
$R^1$, $R^2$, $R^3$, $R^4$, X, Y, m, n, o die oben angegebene Bedeutung haben, und
Hal für Halogen steht.

6. Verfahren zur Herstellung der Verbindungen der Formel IV, dadurch gekennzeichnet, daß man

a) Verbindungen der Formel VI

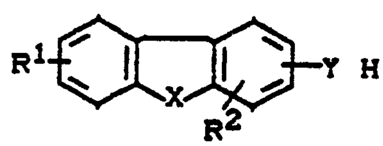

VI

in welcher
$R^1$, $R^2$, X, Y die oben angegebene Bedeutung haben,
mit 1,n-Dihalogenverbindungen der Formel IX

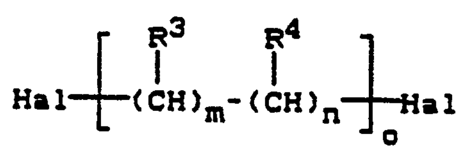

IX

in welcher
Hal, $R^3$, $R^4$, m, n, o die oben angegebene Bedeutung haben,
umsetzt, oder daß man

b) Verbindungen der Formel II

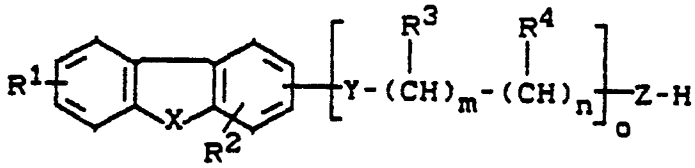

II

in welcher
$R^1$, $R^2$, $R^3$, $R^4$, X, Y, Z, m, n, o die oben angegebene Bedeutung haben,
mit Halogenierungsmitteln umsetzt.

7. Verwendung von substituierten tricyclischen Verbindungen der allgemeinen Formel I zur Bekämpfung von Insekten, insbesondere von Flöhen.

8. Mittel zur Bekämpfung von Insekten, insbesondere von Flöhen, gekennzeichnet durch einen Gehalt an mindestens einer substituierten tricyclischen Verbindung der Formel I, gemäß Anspruch 1.

9. Verfahren zur Bekämpfung von Insekten, insbesondere von Flöhen, dadurch gekennzeichnet, daß man substituierte tricyclische Verbindungen der Formel I gemäß Anspruch 1 auf Flöhe, ihre Wirtstiere und/oder ihren Lebensraum einwirken läßt.

10. Verfahren zur Herstellung von Mitteln zur Bekämpfung von Insekten, insbesondere von Flöhen, dadurch gekennzeichnet, daß man substituierte tricyclische Verbindungen der Formel I gemäß Anspruch 1 mit Streckmitteln und/oder oberflächenaktiven Mitteln vermischt.

11. Verwendung von tricyclischen Verbindungen der Formel I gemäß Anspruch 1 zur Herstellung von Bekämpfungsmitteln gegen Insekten, insbesondere Flöhe.